(19) Europäisches Patentamt
European Patent Office
Office européen des brevets

(11) EP 4 178 676 B1

(12) **EUROPEAN PATENT SPECIFICATION**

(45) Date of publication and mention of the grant of the patent:
**15.04.2026 Bulletin 2026/16**

(21) Application number: **21733206.3**

(22) Date of filing: **10.06.2021**

(51) International Patent Classification (IPC):
***A61P 35/00*** *(2006.01)* ***A61P 37/00*** *(2006.01)*
***C07D 471/04*** *(2006.01)* ***A61K 31/437*** *(2006.01)*

(52) Cooperative Patent Classification (CPC):
**C07D 471/04; A61P 35/00; A61P 37/00**

(86) International application number:
**PCT/IB2021/055118**

(87) International publication number:
**WO 2022/008995 (13.01.2022 Gazette 2022/02)**

(54) **N-1 BRANCHED IMIDAZOQUINOLINES, CONJUGATES THEREOF, AND METHODS**

N-1-VERZWEIGTE IMIDAZOCHINOLINE, KONJUGATE DAVON UND VERFAHREN

IMIDAZOQUINOLINES RAMIFIÉES N-1, CONJUGUÉS DE CELLES-CI ET PROCÉDÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2020 US 202062705633 P**

(43) Date of publication of application:
**17.05.2023 Bulletin 2023/20**

(73) Proprietor: **Solventum Intellectual Properties Company**
**Maplewood, MN 55144 (US)**

(72) Inventors:
- **GRIESGRABER, George W.**
  **Saint Paul, Minnesota 55133-3427 (US)**
- **TOMAI, Mark A.**
  **Saint Paul, Minnesota 55133-3427 (US)**
- **COHEN, Hannah C.**
  **Saint Paul, Minnesota 55133-3427 (US)**
- **HUNERDOSSE, Devon**
  **Saint Paul, Minnesota 55133-3427 (US)**

(74) Representative: **Vossius & Partner Patentanwälte Rechtsanwälte mbB**
**Siebertstraße 4**
**81675 München (DE)**

(56) References cited:
**WO-A1-2012/167081** **WO-A1-2019/166937**
**WO-A1-2020/250089** **US-A1- 2017 217 960**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**Description**

BACKGROUND

Some drug compounds act by stimulating certain key aspects of the immune system, as well as by suppressing certain other aspects. These compounds are sometimes referred to as immune response modifiers (IRMs). Some IRM compounds are useful for treating viral diseases, neoplasias, and $T_H2$-mediated diseases. Some IRM compounds are useful as vaccine adjuvants.

IRM compounds have been reported based on the following bicyclic and tricyclic ring systems: 1H-imidazo[4,5-c]quinolin-4-amines; 1H-imidazo[4,5-c]pyridin-4-amines; 1H-imidazo[4,5-c][1,5]naphthyidin-4-amines; thiazolo[4,5-c]quinolone-4-amines and oxazolo[4,5-c]quinolone-4-amines; 6,7,8,9-1H-tetrahydro-1H-imidazo[4,5-c]quinolin-4-amines; 2H-pyrazolo[3,4-c]quinolone-4-amines; and N-1 and 2-substituted 1H-imidazo[4,5-c]quinolin-4-amines. Conjugation of IRM compounds with polymeric materials or other active compounds is known; however, other conjugates using new IRM compounds with higher activity are desired.

SUMMARY

New compounds that can be useful in inducing cytokine biosynthesis in humans and animals are disclosed. Such compounds (or salts thereof) are of the following Formula (I):

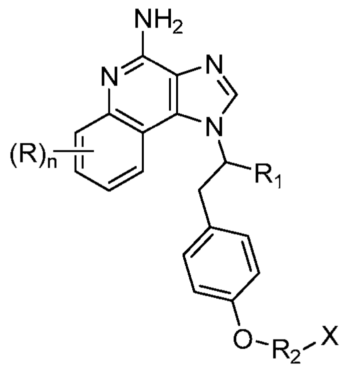

Formula (I),

wherein:

n is an integer of 0 or 1;
R is selected from the group consisting of halogen, hydroxy, alkyl, alkoxy, and -C(O)-O-alkyl;
$R_1$ is $-C_{1-3}$alkylene-O-$C_{1-3}$alkyl;
$R_2$ is a $C_{2-18}$alkylene group or $C_{2-18}$alkenylene group, optionally interrupted by one or more non-peroxidic -O- atoms; and
X is $NH_2$.

The compounds and salts, such as pharmaceutically acceptable salts, of these compounds can be used as immune response modifiers due to their ability to induce cytokine biosynthesis (e.g., induce the synthesis of at least one cytokine) and otherwise modulate the immune response when administered to humans or animals. The compounds can therefore be used in the treatment of a variety of conditions such as viral diseases and tumors that are responsive to such changes in the immune response.

The compounds can also be used in conjugates with polymeric materials or secondary actives. Such conjugates include IRM-containing conjugates of the following Formula (V-A):

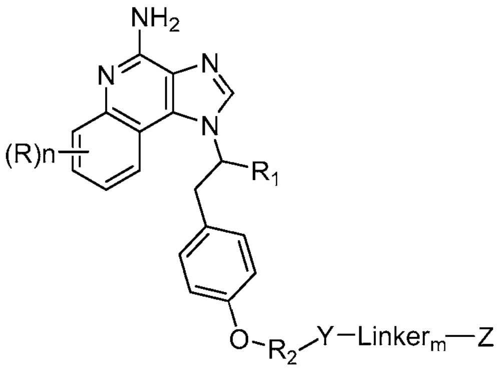

wherein:

n is an integer of 0 or 1;
R is selected from the group consisting of halogen, hydroxy, alkyl, alkoxy, and -C(O)-O-alkyl;
R1 is -$C_{1-3}$alkylene-O-$C_{1-3}$alkyl;
$R_2$ is a $C_{2-18}$alkylene group or $C_{2-18}$alkenylene group, optionally interrupted by one or more non-peroxidic -O- atoms;
Y is N, or NH (which is a residue of X upon bonding with the linker or polymer);
Linker is a heterobifunctional crosslinking group derived from a structure as listed in claim 8;
m = 0 or 1 (i.e., the Linker may or may not be present); and
Z is an antibody.

Pharmaceutical compositions (i.e., formulations) containing an effective amount of a compound (or salt thereof including pharmaceutically acceptable salts thereof) of Formula (I), or an IRM-containing conjugate of Formula (V-A), or a combination thereof, are disclosed. Also disclosed are pharmaceutical compositions for use in inducing cytokine biosynthesis in a human or animal, for treating a viral disease in a human or animal, and for treating a neoplastic disease in a human or animal by administering to the human or animal such formulation.

The term "alkyl" refers to a monovalent group that is a radical of an alkane and includes straight-chain, branched, cyclic, and bicyclic alkyl groups, and combinations thereof. Unless otherwise indicated, the alkyl groups typically contain from 1 to 20 carbon atoms. In some embodiments, the alkyl groups contain 1 to 10 carbon atoms, 1 to 9 carbon atoms, 1 to 8 carbon atoms, 1 to 7 carbon atoms, 1 to 6 carbon atoms, 1 to 5 carbon atoms, 1 to 4 carbon atoms, 1 to 3 carbon atoms, or 1 to 2 carbon atoms. Examples of "alkyl" groups include, but are not limited to, methyl, ethyl, n-propyl, n-butyl, n-pentyl, isobutyl, t-butyl, isopropyl, n-octyl, n-heptyl, ethylhexyl, cyclopentyl, cyclohexyl, cycloheptyl, adamantyl, norbornyl, and the like.

The term "alkylene" refers to a divalent group that is a radical of an alkane and includes groups that are linear, branched, cyclic, bicyclic, or a combination thereof. Unless otherwise indicated, the alkylene group typically has 1 to 20 carbon atoms. In some embodiments, the alkylene group has 2 to 18 carbon atoms, 2 to 14 carbon atoms, 2 to 12 carbon atoms, 2 to 10 carbon atoms, 1 to 10 carbon atoms, 2 to 8 carbon atoms, 2 to 6 carbon atoms, 1 to 6 carbon atoms, 2 to 4 carbon atoms, 1 to 4 carbon atoms, or 1 to 3 carbon atoms. Examples of "alkylene" groups include methylene, ethylene, 1,3-propylene, 1,2-propylene, 1,4-butylene, 1,4-cyclohexylene, and 1,4-cyclohexyldimethylene.

The term "alkenylene" refers to a divalent group that is a radical of an alkene and includes groups that are linear, branched, cyclic, bicyclic, or a combination thereof. Unless otherwise indicated, the alkenylene group typically has 2 to 18 carbon atoms. In some embodiments, the alkenylene group has 2 to 18 carbon atoms, 2 to 14 carbon atoms, 2 to 12 carbon atoms, 2 to 10, 2 to 8 carbon atoms, 2 to 6 carbon atoms, or 2 to 4 carbon atoms. Examples of "alkenylene" groups include ethenylene, propenylene, 1,4-butenylene, 1,4-cyclohexenylene, and 1,4-cyclohexenyldimethylene.

An alkylene or alkenylene group with carbon atoms optionally "interrupted" by one or more non-peroxidic -O- atoms means that the group has carbon atoms on either side of the -O-. Examples include $-CH_2CH_2-O-CH_2CH_2-$, $-CH_2-CH_2-O-CH_2-CH_2-O-CH_2CH_2-$, $-CH_2CH_2-O-CH_2=CH_2-$, $-CH_2-CH_2-O-CH_2=CH_2-O-CH_2CH_2-$, and the like.

The term "alkoxy" refers to a monovalent group having an oxy group bonded directly to an alkyl group.

The term "$C_{x-y}$alkyl," "$C_{x-y}$alkoxy," and $C_{x-y}$alkylene" are inclusive of straight chain groups, branched chain groups, cyclic groups, and combinations thereof that have X to Y carbon atoms. For example, a "$C_{1-5}$alkyl" includes alkyl groups of 1 carbon, 2 carbons, 3 carbons, 4 carbons, and 5 carbons. Some examples of "$C_{1-5}$alkyl" include methyl, ethyl, n-propyl, isopropyl, n-butyl, sec-butyl, isobutyl, isomeric pentyls, cyclopropyl, cyclopentyl, and $-CH_2$-cyclopropyl.

"IRM-containing conjugate" and variations thereof refers to any conjugate (i.e., conjugate) that includes at least one immune response modifier (IRM) moiety derived from the IRM compound of formula (I) and at least one polymeric moiety (e.g., a PEG moiety) or second active moiety.

"Moiety" and variations thereof refer to a portion of a chemical compound or polymer that exhibits a particular character such as, for example, a particular biological or chemical function (e.g., immunomodulation and/or target specificity), or a physical property (e.g., size, hydrophilicity or hydrophobicity).

"Heterobifunctional crosslinking group" is derived from a heterobifunctional crosslinking compound that reacts to forms a first bond with the X group of the IRM compound and a second bond with a reactive group (e.g., hyroxyl (-OH), amino (-$NH_2$), amido (-NHC(O)), aldehyde (-CH(O)), or sulfhydryl (-SH) group) of a polymer or a second active compound. The heterobifunctional crosslinking compound (i.e., heterobifunctional crosslinker) includes two different reactive groups at either end, and an organic cross-bridge of various length and composition.

"Labile bond" refers to a bond that is readily cleaved in vivo so that the link between the IRM moiety and the polymeric moiety or second active moiety is broken, thereby releasing free and active IRM compound of Formula (I) that is capable of contacting immune cells and inducing an immune response.

The "salt" of a compound includes pharmaceutically acceptable salts, such as those described in Berge, Stephen M., "Pharmaceutical Salts," Journal of Pharmaceutical Sciences, 1977, 66, pages 1-19. For example, salts can be prepared by reacting a free base compound (that is, one not in a salt form) with an inorganic or organic acid such as, for example, hydrochloric acid, sulfuric acid, hydrobromic acid, methane sulfonic acid, ethane sulfonic acid, malic acid, maleic acid, acetic acid, trifluoroacetic acid, para-toluenesulfonic acid, salicylic acid, succinic acid, tartaric acid, citric acid, pamoic acid, xinafoic acid, oxalic acid, and the like.

As used herein, "pharmaceutically acceptable carriers" include those carriers that can deliver therapeutically or prophylactically effective amounts of one or more of the compounds, salts, or conjugates of the disclosure to a subject by a chosen route of administration, are generally tolerated by the subject, and have an acceptable toxicity profile (preferably minimal to no toxicity at an administered dose). Some suitable pharmaceutically acceptable carriers are described in Remington’s Pharmaceutical Sciences, 18th Edition (1990), Mack Publishing Co. and can be readily selected by one of ordinary skill in the art. Typical pharmaceutically acceptable salts include hydrochloride and dihydrochloride.

"Effective amount" (including "therapeutically effective amount" and "prophylactically effective amount") are defined as an amount of compound, salt, or conjugate sufficient to induce a therapeutic or prophylactic effect, such as cytokine induction, immunomodulation, antitumor activity, and/or antiviral activity. Depending on the disease or condition, the desired cytokine profile, and/or the acceptable level of side effects, the effective amount may vary. For example, a small amount of a very active compound or salt, or a large amount of a compound or salt of low activity, may be used to avoid undesirable side effects.

"Treat" and "Treatment" as well as variations thereof refer to reducing, limiting progression, ameliorating, preventing, or resolving to any extent the symptoms or signs related to a condition. "Ameliorate" and "ameliorating" refers to any reduction in the extent, severity, frequency, and/or likelihood of a symptom or clinical characteristic of a particular disease or condition.

Herein, the term "comprises" and variations thereof do not have a limiting meaning where these terms appear in the description and claims. Such terms will be understood to imply the inclusion of a stated step or element or group of steps or elements but not the exclusion of any other step or element or group of steps or elements. By "consisting of" is meant including, and limited to, whatever follows the phrase "consisting of." Thus, the phrase "consisting of" indicates that the listed elements are required or mandatory, and that no other elements may be present. By "consisting essentially of" is meant including any elements listed after the phrase, and limited to other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they materially affect the activity or action of the listed elements. Any of the elements or combinations of elements that are recited in this specification in open-ended language (e.g., comprise and derivatives thereof), are considered to additionally be recited in closed-ended language (e.g., consist and derivatives thereof) and in partially closed-ended language (e.g., consist essentially, and derivatives thereof).

The words "preferred" and "preferably" refer to embodiments of the disclosure that may afford certain benefits, under certain circumstances. However, other embodiments may also be preferred, under the same or other circumstances. Furthermore, the recitation of one or more preferred embodiments does not imply that other embodiments are not useful, and it is not intended to exclude other embodiments from the scope of the disclosure.

In this application, terms such as "a," "an," and "the" are not intended to refer to only a singular entity, but include the general class of which a specific exan ple may be used for illustration. The terms "a," "an," and "the" are used interchangeably with the term "at least one." The phrases "at least one of" and "comprises at least one of" followed by a list refers to any one of the items in the list and any combination of two or more items in the list.

As used herein, the term "or" is generally employed in its usual sense including "and/or" unless the content clearly dictates otherwise.

The term "and/or" means one or all of the listed elements or a combination of any two or more of the listed elements.

Also herein, all numbers are assumed to be modified by the term "about" and in certain embodiments, preferably,

by the term "exactly." As used herein in connection with a measured quantity, the term "about" refers to that variation in the measured quantity as would be expected by the skilled artisan making the measurement and exercising a level of care commensurate with the objective of the measurement and the precision of the measuring equipment used. Herein, "up to" a number (e.g., up to 50) includes the number (e.g., 50).

Also herein, the recitations of numerical ranges by endpoints include all numbers subsumed within that range as well as the endpoints (e.g., 1 to 5 includes 1, 1.5, 2, 2.75, 3, 3.80, 4, 5, etc.).

As used herein, the terms "ambient temperature" or "room temperature" refers to a temperature of 20°C to 25°C or 22°C to 25°C.

The term "in the range" or "within a range" (and similar statements) includes the endpoints of the stated range.

Groupings of alternative elements or embodiments disclosed herein are not to be construed as limitations. Each group member may be referred to and claimed individually or in any combination with other members of the group or other elements found therein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

When a group is present more than once in a formula described herein, each group is "independently" selected, whether specifically stated or not. For example, when more than one R group is present in a formula, each R group is independently selected.

Reference throughout this specification to "one embodiment," "an embodiment," "certain embodiments," or "some embodiments," etc., means that a particular feature, configuration, composition, or characteristic described in connection with the embodiment is included in at least one embodiment of the invention. Thus, the appearances of such phrases in various places throughout this specification are not necessarily referring to the same embodiment of the invention. Furthermore, the particular features, configurations, compositions, or characteristics may be combined in any suitable manner in one or more embodiments.

The above summary of the present disclosure is not intended to describe each disclosed embodiment or every implementation of the present invention. The description that follows more particularly exemplifies illustrative embodiments. In several places throughout the application, guidance is provided through lists of examples, which examples may be used in various combinations. In each instance, the recited list serves only as a representative group and should not be interpreted as an exclusive list. Thus, the scope of the present disclosure should not be limited to the specific illustrative structures described herein, but rather extends at least to the structures described by the language of the claims. Any of the elements that are positively recited in this specification as alternatives may be explicitly included in the claims or excluded from the claims, in any combination as desired. Although various theories and possible mechanisms may have been discussed herein, in no event should such discussions serve to limit the claimable subject matter.

## DETAILED DESCRIPTION OF ILLUSTRATIVE EMBODIMENTS

The invention is as described in the appended claims. Embodiments outside of the scope of the claims are provided for reference purposes only.

### IRM Compounds

This disclosure provides compounds (or salts thereof) of the following Formula (I):

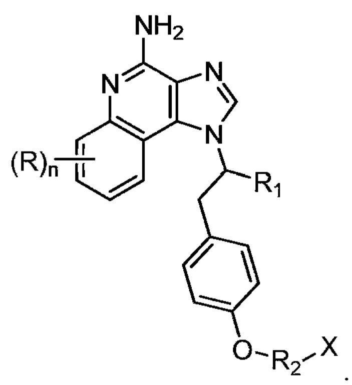

In certain embodiments of Formula (I), n is an integer of 0 or 1. In certain embodiments of Formula (I), n is 0.

In certain embodiments of Formula (I), R is selected from the group consisting of halogen, hydroxy, alkyl, alkoxy,

and -C(O)-O-alkyl. In certain embodiments of Formula (I), R is selected from the group consisting of halogen, hydroxy, alkyl, and alkoxy. In certain embodiments of Formula (I), R is selected from the group consisting of halogen, hydroxy, $-C_{1-7}$alkyl, and $-C_{1-7}$alkoxy. In certain embodiments of Formula (I), R is selected from the group consisting of hydroxy, F, and Cl. In certain embodiments of Formula (I), R is selected from the group consisting of F and Cl.

In certain embodiments of Formula (I), R1 is -C1-3alkylene-O-C1-3alkyl. In certain embodiments of Formula (I), $R_1$ is $-CH_2OCH_3$ or $-CH_2OCH_2CH_3$. In certain embodiments of Formula (I), $R_1$ is $-CH_2OCH_2CH_3$.

In certain embodiments of Formula (I), $R_2$ is a $C_{2-18}$alkylene group or $C_{2-18}$alkenylene group, optionally interrupted by one or more non-peroxidic -O- atoms. In certain embodiments of Formula (I), $R_2$ is a $C_{2-18}$alkylene group optionally interrupted by one or more non-peroxidic -O-atoms. In certain embodiments of Formula (I), $R_2$ is a $C_{2-12}$alkylene group. In certain embodiments of Formula (I), $R_2$ is a $C_{2-6}$alkylene group. In certain embodiments of Formula (I), $R_2$ is a $C_{2-4}$alkylene group.

In certain embodiments of Formula (I), X is OH or $NH_2$. In certain embodiments of Formula (I), X is OH. In certain embodiments of Formula (I), X is $NH_2$.

In certain embodiments, the compound is of Formula (II), or a salt thereof:

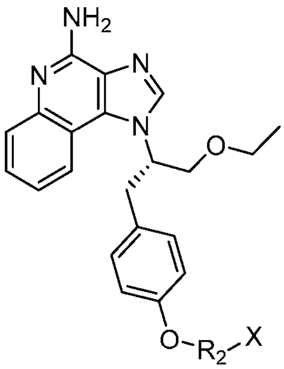

In certain embodiments of Formula (II), $R_2$ is a $C_{2-18}$alkylene group or $C_{2-18}$alkenylene group, optionally interrupted by one or more non-peroxidic -O- atoms. In certain embodiments of Formula (II), $R_2$ is a $C_{2-18}$alkylene group optionally interrupted by one or more non-peroxidic -O-atoms. In certain embodiments of Formula (II), $R_2$ is a $C_{2-12}$alkylene group. In certain embodiments of Formula (II), $R_2$ is a $C_{2-6}$alkylene group. In certain embodiments of Formula (II), $R_2$ is a $C_{2-4}$alkylene group.

In certain embodiments of Formula (II), X is OH or $NH_2$. In certain embodiments of Formula (II), X is $NH_2$.

In certain embodiments, the compound is of Formula (III), or salt thereof:

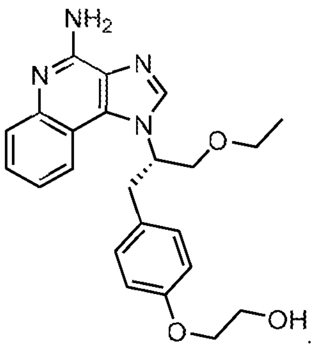

In certain embodiments, the compound is of Formula (IV), or salt thereof:

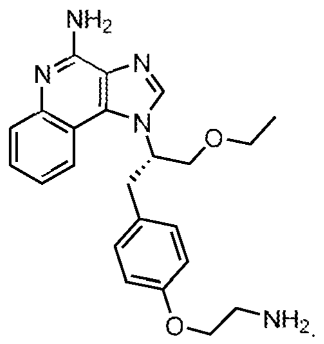

Preparation of IRM Compounds

The compounds of the disclosure may be synthesized by synthetic routes that include processes analogous to those well known in the chemical arts, particularly in light of the description contained herein. The starting materials are generally available from commercial sources such as the Sigma-Aldrich Company (St. Louis, MO) or are readily prepared using methods well known to those of ordinary skill in the art (e.g., prepared by methods generally described in Louis F. Fieser and Mary Fieser, Reagents for Organic Synthesis, v. 1-26, Wiley, New York; Alan R. Katritsky, Otto Meth-Cohn, Charles W. Rees, Comprehensive Organic Functional Group Transformations, v 1-6, Pergamon Press, Oxford, England, (1995); Barry M. Trost and Ian Fleming, Comprehensive Organic Synthesis, v. 1-8, Pergamon Press, Oxford, England, (1991); or Beilsteins Handbuch der Organischen Chemie, 4, Aufl. Ed. Springer-Verlag, Berlin, Germany, including supplements (also available via the Beilstein online database)).

Compounds of the disclosure can be prepared, for example, according to Reaction Schemes I and II where R, R1, R2, X and n are as described above. In step (1) of Reaction Scheme I, (S)-2-(tert-butoxycarbonylamino)-3-(4-tert-butoxyphenyl)propanoic acid of Formula (VI) (a di-protected version of tyrosine) can be can be reacted with isobutyl chloroformate and N-methyl morpholine followed by reaction with sodium borohydride in step (2) to provide the alcohol of Formula (VII). Alkylation of the alcohol of Formula (VII) in step (3) with an alkylating agent such as for example dialkylsulfate or an alkyl halide can provide the alkyl ether of Formula (VIII). In step (4) of Reaction Scheme I, the protecting groups can be removed from the compound of Formula (VIII) using concentrated hydrochloric acid in ethanol with heating to provide the compound of Formula (IX).

Reaction Scheme I

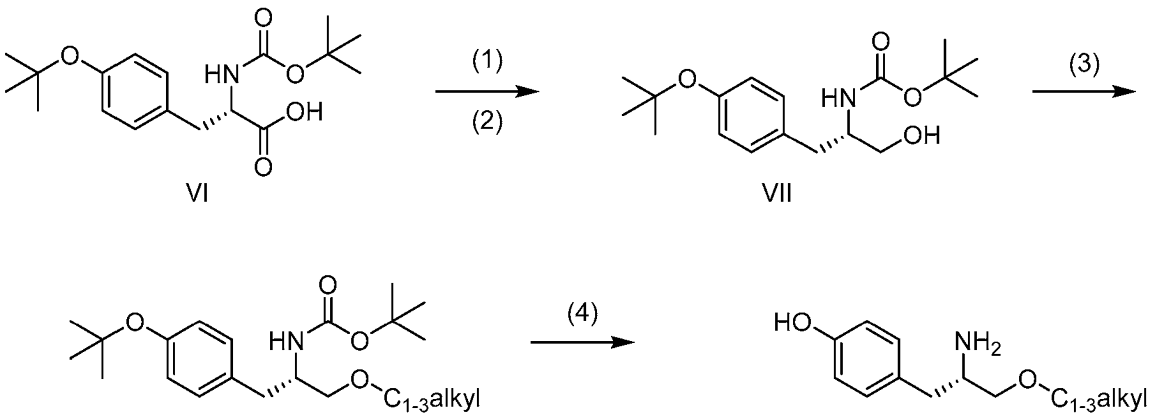


VI VII VIII IX

In Reaction Scheme II, a 4-chloro-3-nitroquinoline of Formula (X) is reacted in step (5) with the compound of Formula (IX) to provide a 3-nitroquinolin-4-amine of Formula (XI). The reaction can be carried out by adding the amine of Formula (IX) to a solution of Formula (X) in a suitable solvent such as dichloromethane in the presence of a tertiary amine such as triethylamine. The 4-chloro-3-nitroquinoline compound of Formula (X) and substituted analogs are known compounds (see, for example, U.S. Patent Numbers 3,700,674 (Diehl et al.), 5,389,640 (Gerster et al.), 6,110,929 (Gerster et al.), 7,923,560 (Wightman et al.), and references cited therein). In many cases, substituted analogs of Formula (X) (for example, n = 1 and R being a halogen, alkoxy, or benzyloxy group) can be prepared starting with commercially available substituted anilines.

In step (6) of Reaction Scheme II, the phenoxy group of Formula (XI) can be converted to an ether of Formula (XII) using conventional synthetic methods. For example, the compound of Formula (XI) can be reacted with a suitable alkylating agent of formula LG-$R_2$-Y-PG and a base (such as cesium carbonate) in an inert solvent (such as N,N-dimethylformamide) where LG is a leaving group, Y is -O- or -NH-, PG is a protecting group and $R_2$ is as defined above. Suitable leaving groups include, but are not limited to, bromide, iodide, methanesulfonyloxy and p-toluenesulfonyloxy. Suitable protecting groups when Y = -O- include, but are not limited to, acetyl, benzyl, tetrahydropyranyl and silyl protecting groups (e. g. trimethylsilyl, triethylsilyl, tert-butyl dimethylsilyl and the like). Suitable protecting groups when Y = -NH- include, but are not limited to, tert-butoxycarbonyl (BOC) and benzyloxycarbonyl (CBZ). Suitable alkylating agents are commercially available (2-bromoethyl acetate and 2-(Boc-amino)ethyl bromide) or can be prepared using conventional synthetic methods.

In step (7) of Reaction Scheme II, the nitro group of Formula (XII) can be reduced to an amino group. The reduction can be carried out in a pressure bottle using hydrogen, a catalytic amount of palladium or platinum on carbon, and a solvent such as methanol, acetonitrile, toluene, or combinations thereof. The reaction can be carried out with a Parr apparatus. In step (8) of Reaction Scheme II, the resulting 3,4-diamine compound can be reacted with a triethyl orthoformate to provide a 1H-imidazo[4,5-c]quinoline of Formula (XIII). The reaction can be carried out an inert solvent such as propyl acetate or toluene. Optionally, a catalyst such as pyridine hydrochloride can be included.

In step (9) of Reaction Scheme II, the 1H-imidazo[4,5-c]quinoline of Formula (XIII) can be oxidized to provide a 1H-imidazo[4,5-c]quinoline-5N-oxide using a conventional oxidizing agent capable of forming an N-oxide. Preferably, a solution of the compound of Formula (XIII) in a suitable solvent such as chloroform or dichloromethane is reacted with 3-chloroperbenzoic acid at ambient temperature.

In step (10) of Reaction Scheme II, the N-oxide compound can be aminated to provide a 1H-imidazo[4,5-c]quinoline-4-amine of Formula (XIV). Step (10) involves reacting the N-oxide compound with a sulfonylating agent and an aminating agent in an inert solvent such as dichloromethane or chloroform. Suitable sulfonylating agents include alkyl- or arylsulfonyl chlorides such as benzenesulfonyl chloride, methanesulfonyl chloride, or para-toluenesulfonyl chloride. Ammonium hydroxide is a suitable aminating agent.

In step (11) of Reaction Scheme II, the protecting group (PG) of Formula (XIV) can be removed to give a compound of Formula (XV). For example, when Y = -O- and PG is an acetyl, the compound of Formula (XIV) can be reacted with a suitable base, such as sodium methoxide, to remove the acetyl protecting group to provide a compound of Formula (XV) where X = OH. In another example, where Y = -NH- and PG is a tert-butoxycarbonyl, the compound of Formula (XIV) can be reacted with hydrochloric acid in an alcoholic solvent to remove the tert-butoxycarbonyl group to provide a compound of Formula (XV) where X = $NH_2$. Formula (XV) is an embodiment of Formula (I).

Reaction Scheme II

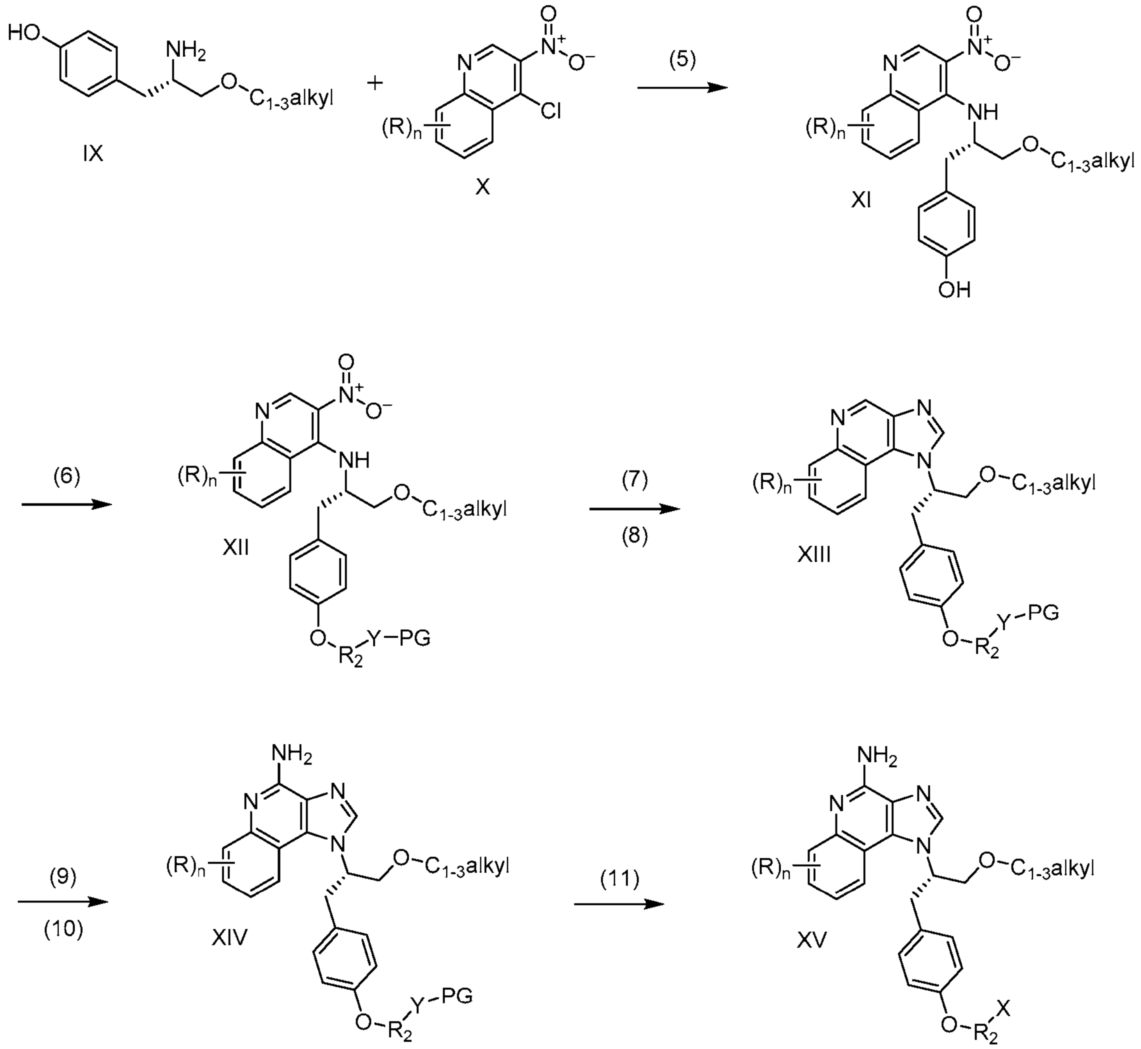


IX X XI XII XIII XIV XV

Compounds of the disclosure can be prepared according to Reaction Schemes I and II with the starting compound of Formula (VI) being replaced with similarly di-protected versions of (S)-3-amino-4-(4-hydroxyphenyl) butanoic acid and (S)-4-amino-5-(4-hydroxyphenyl)pentanoic acid.

In the preparation of the compounds of the disclosure it is understood by one of ordinary skill in the art that it may be necessary to protect a particular functional group while reacting other functional groups of an intermediate compound. The need for such protection will vary depending on the nature of the particular functional group and the conditions of the particular reaction step. A review of reactions for protecting and deprotecting functional groups can be found in P.G.M. Wuts, Greene's Protective Groups in Organic Synthesis, John Wiley & Sons, 20 New York, USA, 2014.

Conventional methods and techniques of separation and purification can be used to isolate the IRM compounds used in the compositions of the disclosure. Such techniques may include, for example, all types of chromatography (high performance liquid chromatography (HPLC), column chromatography using common absorbents such as silica gel, and thin layer chromatography), recrystallization, and differential (i.e., liquid-liquid) extraction techniques.

Compounds described herein may be in the form of enantiomers. The enantiomeric excess of the compounds, or salts thereof, of the disclosure can be determined using standard analytical assays such as gas chromatography or HPLC with a column having a chiral stationary phase (CSP). Suitable columns with a CSP are available from Chiral Technologies, Inc., Westchester, PA.

Enantiomeric excess (% ee) is calculated according to Equation 1.

Equation 1.

$$\text{enantiomeric excess (\% ee)} = \frac{\left(\begin{array}{c}\text{mole \% of}\\ \text{major enantiomer}\end{array}\right) - \left(\begin{array}{c}\text{mol \% of}\\ \text{minor enantiomer}\end{array}\right)}{\left(\begin{array}{c}\text{mole \% of}\\ \text{major enantiomer}\end{array}\right) + \left(\begin{array}{c}\text{mole \% of}\\ \text{minor enantiomer}\end{array}\right)} \text{X } 100$$

Enantiomeric excess (% ee) can be calculated from a chiral HPLC chromatogram by comparing the peak areas of the major enantiomer and minor enantiomer signals according to Equation 2.

Equation 2.

$$\text{enantiomeric excess (\% ee)} = \frac{\left(\begin{array}{c}\text{peak area of}\\ \text{major enantiomer}\end{array}\right) - \left(\begin{array}{c}\text{peak area of}\\ \text{minor enantiomer}\end{array}\right)}{\left(\begin{array}{c}\text{peak area of}\\ \text{major enantiomer}\end{array}\right) + \left(\begin{array}{c}\text{peak area of}\\ \text{minor enantiomer}\end{array}\right)} \text{X } 100$$

IRM-Containing Conjugates

This disclosure provides IRM-containing conjugates of the following Formula (V-A):

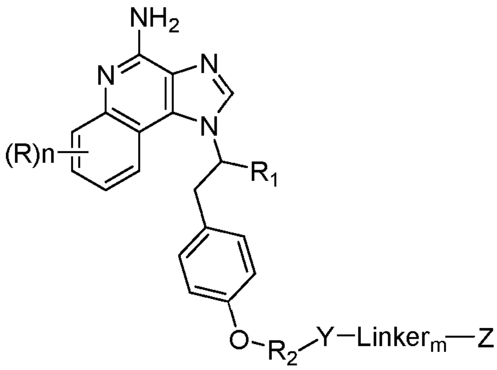

In the disclosure of Formula (V-A), n, R, R1, and $R_2$ are as described above for Formula (I).

In certain embodiments of Formula (V-A), Y is O, N, or NH. Y is a residue of the group X in Formula (I) upon bonding with a linking group to form the linker, or with a polymer or secondary active compound. In certain embodiments of Formula (V-A), Y is O. In certain embodiments of Formula (V-A), Y is NH. In certain embodiments of Formula (V-A), Y is N.

In certain embodiments of Formula (V-A), Linker is a heterobifunctional crosslinking group.

In certain embodiments of Formula (V-A), m = 0 or 1 (i.e., the Linker may or may not be present). The Linker, if present (when m = 1), is derived from a heterobifunctional compound.

In certain embodiments of Formula (V-A), Z is a polymeric moiety or second active moiety.

In certain embodiments of Formula (V-A), the Y-$\text{Linker}_m$-Z portion of the conjugate, with or without a linker, optionally includes a labile bond.

This disclosure provides IRM-containing conjugates of the following Formula (V-B):

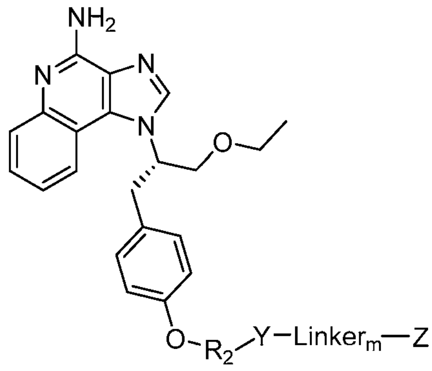

In the disclosure of Formula (V-B), $R_2$ Y, Linker, m, and Z are as described above for Formula (V-A).

Linker of IRM-Containing Conjugates

The Linker, which is present when m = 1, is derived from a heterobifunctional compound.

An IRM-containing conjugate (i.e., IRM-containing complex) of Formula (V-A) can be prepared using a heterobifunctional crosslinker. The general definition of a heterobifunctional crosslinker is as follows " ... a heterobifunctional cross-linking agent includes two different reactive groups at either end, and an organic cross-bridge of various length and composition." (Hermanson, G. (1996), Bioconjugate Techniques, Academic Press, Chapter 5 "Heterobifunctional Cross-Linkers", page 229). For example, compounds of Formula (I) can be reacted with a heterobifunctional crosslinker to form ester or amide bonds. The other reactive group(s) on the heterobifunctional crosslinker are chosen so that they can react with functional groups on the Z component of the IRM-containing complex of Formula (V-A). Useful functional groups often found on the Z component of the IRM-containing complex of Formula (V-A) include, but are not limited to, amines ($-NH_2$), thiols (-SH), and aldehydes (-CHO), which can be derivatized with heterobifunctional crosslinkers that contain, respectively, amine reactive groups, thiol reactive groups, and aldehyde reactive groups.

Many heterobifunctional crosslinkers are known (Hermanson, G. (1996), Bioconjugate Techniques, Academic Press, Chapter 5 "Heterobifunctional Cross-Linkers", pages 229-285) and many are commercially available (Thermo-Fisher Scientific Incorporated, Waltham MA and other suppliers). In addition, heterobifunctional crosslinkers can also be synthesized using conventional methods.

Heterobifunctional crosslinkers of the present invention are: sulfo-N-succinimidyl 4-(maleimidomethyl)cyclohexane-1-carboxylate, sodium salt (Sulfo SMCC)

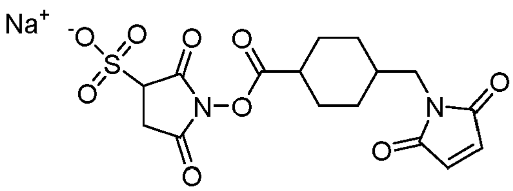

N-(γ-maleimidobutyryloxy)sulfosuccinimide ester (Sulfo-GMBS)

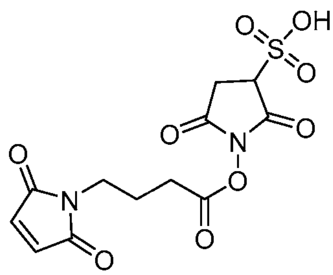

succinimidyl-[(N-maleimidopropionamido)-tetraethyleneglycol]ester (NHS-$PEO_4$-Maleimide)

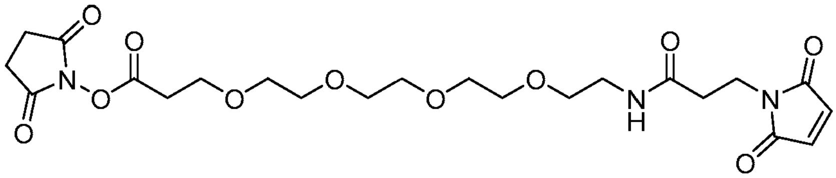

N-succinimidyl 3-(bromoacetamido)propionate (SBAP)

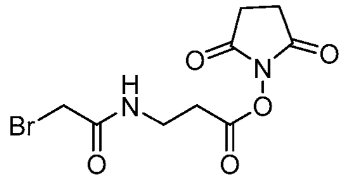

and 4-succinimidyloxycarbonyl-methyl-α-(2-pyridyldithio)toluene (SMPT)

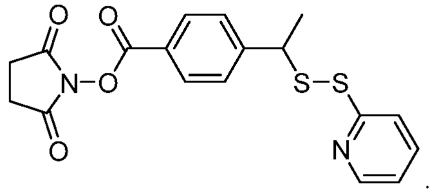

Optional Labile Bond in IRM-Containing Conjugates

In certain embodiments of Formula (V-A), the Y-Linker$_m$-Z portion of the conjugate, with or without a linker, optionally includes a labile bond. A "labile bond" refers to a bond that is readily cleaved in vivo so that the link between the IRM moiety and the polymeric moiety or second active moiety is broken, thereby releasing free and active IRM compound of Formula (I) that is capable of contacting immune cells and inducing an immune response, as well as a second active in certain embodiments.

The labile bond may be any covalent bond that is readily cleaved in vivo and that links the second active moiety or polymeric moiety to the IRM moiety at a location on the IRM moiety that causes a substantial reduction in the immunomodulatory activity of the IRM moiety. When the labile bond is intact-i.e., when the IRM moiety is linked to the second active moiety or polymeric moiety-the IRM moiety may have substantially reduced immunomodulatory activity. When the labile bond is cleaved, however, a free and active IRM moiety-now a free IRM compound-is released and capable of inducing an immune response.

In some cases, the reduction in immunomodulatory activity may be due primarily to the identity and nature-e.g., size and/or steric nature-of the substitution. In these cases, the substitution may reduce the immunomodulatory activity of the IRM moiety by, for example, covering the portion of the IRM moiety that binds to receptors and initiates a cell signaling cascade that results in an immune response.

Examples of suitable labile bonds include, but are not limited to, an amide bond, a carbamate bond, an amidine bond, an ester bond, a disulfide bond, or the amide bond of a peptide unit used with or without a self-immolative spacer, such as those described in the literature (Toki, B. E. et al., J. Org. Chem., 2002, 67, 1866-1872; Jeffrey, S. C. et al., J. Med. Chem., 2005, 48, 1344-1358; Sun, M. M. C. et al., Bioconjugate Chem. 2005, 16, 1282-1290), Tsuchikama, K. and An, Z. Protein Cell 2018, 9, 33-46 and International Publication No. WO 2005/082023 (Genentech, Inc.).

In some embodiments, the labile bond is selected from the group consisting of an amide bond, a carbamate bond, an amidine bond, an ester bond, and a disulfide bond. In other embodiments, the labile bond is selected from the group consisting of an amide bond, a carbamate bond, and an amidine bond. In some embodiments, the labile bond is an amide bond.

The labile bond is readily cleaved in vivo. The cleavage may occur by various mechanisms, such as through a chemical (e.g., hydrolysis at physiological pH or hydrolysis at the lower pH environment found within certain tumors) or enzymatic (e.g., reaction with an esterase) biotransformation.

For example, conjugates designed for use treating tumors may include a tumor-specific targeting moiety and a labile bond that is selected because it is more likely, more quickly, or more efficiently cleaved in a tumor environment than in the systemic environment. The microenvironment of tumors is often characterized as having low oxygen tension, low extracellular pH, and low glucose concentration. Labile bonds that can exploit one or more of these microenvironmental

conditions, e.g., low pH, may make a labile bond particularly well suited for use in a conjugate designed for treating the tumor.

Polymeric Moieties of IRM-Containing Conjugates

In certain embodiments of Formula (V-A), Z is a polymeric moiety (i.e., the IRM-containing conjugate is an IRM-polymer conjugate). In certain embodiments of Formula (V-A), the polymeric moiety is derived from a wide variety of polymers.

Suitable polymers may be based on biopolymers or naturally occurring monomers and combinations thereof. Natural biopolymers may include single or double stranded RNA or DNA, comprised of nucleotides (e.g., adenosine, thymidine). The natural biopolymers can be peptides comprised of amino acids. A specific example of this is poly(lysine). Biopolymers can be polysaccharides, which may include but are not limited to, glycogen, cellulose, and dextran. Additional examples include polysaccharides that occur in nature, including alginate and chitosan. Suitable polymers may also be comprised of naturally occurring small molecules, such as lactic acid or glycolic acid, or may be a copolymer of the two (i.e., PLGA). Suitable preformed particles may also be based on formulations (e.g., stabilized emulsions, liposomes and polymersomes) or may be mineral salts that form particles suitable for conjugation or ion exchange on the surfaces of the particles, which may include Aluminum-based salts.

In certain embodiments, the polymer is selected from polyethylene glycol (PEG), glycogen, cellulose, dextran, alginate, chitosan, polylactide, and combinations thereof. In certain embodiments, the polymer is PEG.

The following description of PEG applies to other polymers that form the polymeric moieties of the IRM-containing conjugates.

The PEG moiety may be, or be derived from, any suitable PEG polymer. In some cases, the resulting IRM-PEG conjugate possesses a molecular weight of at least 16 kilodaltons (kDa). In some embodiments, the resulting IRM-PEG conjugate may possess a molecular weight of at least 20 kDa. In other embodiments, the IRM-PEG conjugate has a molecular weight of at least 30 kDa.

In many embodiments, the IRM-PEG conjugate has a molecular weight of no greater than 500 kilodaltons (kDa). In some embodiments the IRM-PEG conjugate has a molecular weight of no greater than 200 kDa. In certain embodiments, the IRM-PEG conjugate has a molecular weight of no greater than 100 kDa, and often no greater than 50 kDa.

Various possible PEG polymers, and methods for attaching the PEG polymers to an IRM compound, are described for example, in International Patent Publication No. WO 2005/110013 (3M).

Some PEG polymers may include a plurality of sites at which an IRM moiety may be attached. Thus, an IRM-PEG conjugate may include a plurality of IRM moieties. In such cases, the plurality of IRM moieties may be homogeneous (i.e., derived from the same IRM compound) or may be heterogeneous (i.e., derived from different IRM compounds).

An IRM-PEG conjugate can provide active, or potentially active, IRM compound to a localized tissue region and/or tissue type, while reducing overall systemic activity of the IRM. In some cases, the IRM-PEG conjugate may be of a size and chemical nature to allow preferential deposition in tissues (e.g., particular tissue types or localized tissue regions) such as solid tumors. This can occur as a result of the tissue's increased vascular permeability, for example, to an IRM-PEG conjugate and the reduced lymphatic drainage of tumor tissues.

One or more IRM moieties can be attached to a PEG moiety through either covalent attachment or non-covalent attachment. Non-covalent attachment of an IRM moiety to a macromolecule moiety includes, for example, affinity attachment (e.g., avidin-biotin).

Representative methods for covalently attaching an IRM moiety to a PEG moiety include chemical crosslinkers, such as heterobifunctional crosslinking compounds that react to form a bond between a reactive group (such as hydroxyl, amino, amido, or sulfhydryl groups) in an immune response modifier and other reactive groups (of a similar nature) in the PEG. This bond may be, for example, a peptide bond, disulfide bond, thioester bond, amide bond, thioether bond, and the like. IRM compounds can also be covalently attached to a PEG by reacting an IRM containing a reactive group directly with a polymer containing a reactive group. Methods for attaching an IRM moiety to a PEG moiety are described in detail in, for example, International Patent Publication No. WO2005/110013 (3M).

Regardless of the particular method used to couple the IRM moiety and the PEG moiety, the link may be cleaved by, for example, hydrolysis or enzymatic activity to yield free IRM compound.

In embodiments in which the IRM-PEG conjugate provides an IRM prodrug, cleavage of the link between the IRM moiety and the PEG moiety may be controlled to some extent. For example, the link may be designed to be hydrolyzed in a particular biological microenvironment. The extracellular environment of tumors is known to be more acidic than the extracellular environment of normal tissues. Thus, the IRM-PEG conjugate may be designed as a prodrug in which the link between the IRM moiety and the PEG moiety remains intact at normal tissue extracellular pH (7.4-7.5), but is hydrolyzed in a solid tumor extracellular pH (less than 7.2). Thus, a pharmaceutical composition that includes an IRM-PEG conjugate and an anti-tumor antigen may be administered in the vicinity of a solid tumor. The IRM-PEG conjugate and antigen can infiltrate the tumor environment (e.g., by diffusion from the thermoresponsive gel carrier) where the IRM-PEG conjugate is

cleaved to yield free IRM. This results in the co-localization of anti-tumor antigen and free IRM that can be co-delivered to immune cells in the vicinity of the tumor, thereby generating an antigen-specific, and therefore tumor-specific, immune response.

In other embodiments, the link between the IRM moiety and the PEG moiety may be designed so that the link is not cleaved unless and until the conjugate reaches the endosomes of an immune cell (e.g., an antigen presenting cell such as a dendritic cell).

The size and structure of the PEG moiety may influence the kinetics under which the link between the IRM moiety and the PEG moiety is cleaved. For example, a PEG moiety may include a poly-armed PEG. The number and size of the PEG arms may influence the kinetics of enzymatic cleavage of the IRM-PEG linkage, thereby releasing free IRM. As another example, the nature of the link between the IRM moiety and the PEG moiety can impact on the rate at which the link is cleaved by hydrolysis. Amide linkages tend to be more readily hydrolyzed than carbamate linkages.

Second Active Moieties of IRM-Containing Conjugates

In certain embodiments of Formula (V-A), Z is a second active moiety (SAM) (i.e., the IRM-containing conjugate is an IRM-SAM conjugate).

The second active moiety may be any moiety other than a second IRM moiety that possesses a biological activity. For example, the second active moiety may include an antigen or a targeting moiety.

Conjugates that include an antigen and an active IRM moiety are described, for example, in U.S. Patent Publication No. 2004/091491 (Kedl et al.). These conjugates can increase the immune response against the antigen by promoting the co-delivery of IRM compound and antigen to an antigen presenting cell.

Some embodiments of the present disclosure include conjugates that include an antigen and an IRM moiety in which the IRM moiety is inactive until the labile bond is cleaved, releasing an active IRM moiety. Such conjugates may be useful for allowing an administered conjugate to reach a target tissue before inducing an immune response. This may provide a therapeutic benefit by inducing a more highly localized antigen-specific immune response. The IRM moiety may be kept inactive until the conjugate reaches the targeted tissue where the antigen-specific immunotherapy is needed, thereby reducing, even preventing, a systemic immune response against the antigen that could be induced by an active IRM moiety before the conjugate is able to reach its target tissue.

In certain embodiments, the second active moiety may be a targeting moiety-i.e., a moiety that acts to target the delivery, or cause the selective retention, of the conjugate to a particular tissue or cell population. The particular nature of a targeting moiety may be determined, to some extent, by the identity and nature of the intended target. For example, a suitable targeting moiety may actively provide directed binding to a target, as in an antibody directed against the antigenic portion of a tumor, target cell, target tissue, or target organ. Active targeting can also be achieved by exploiting receptor-ligand affinity. In other cases, the targeting moiety may provide passive retention of the conjugate in a target. Passive retention may be accomplished by exploiting differences in hydrophobicity/hydrophilicity, vascular porosity, etc. of target vs. non-target environments.

A targeting moiety may be any material that can provide targeted delivery of a conjugate. In many embodiments, the targeting portion may provide immunospecific targeting, i.e., may be a sufficient portion of an immunoglobulin (i.e., an antibody) to promote immunospecific binding of the composition to a target antigen. However, aspects of the present disclosure may be practiced using non-immunoglobulin targeting materials as well such as, for example, receptor ligands such as, for example, hormones (natural or synthetic), lipids, etc.

In some cases, a targeting moiety may be an antibody or be derived from an antibody (i.e., at least enough of the immunospecific portion of an antibody-e.g., enough of a light chain-to provide some degree of immunospecificity. However, in other cases, a targeting moiety may be, or be derived from, an agent that recognizes at least a portion of a tumor-specific marker such as, for example, a ligand that binds to a receptor that is, to some extent, specifically expressed by the target cell population. In such a case, the receptor may be considered a tumor-specific marker.

Conjugates designed for use treating tumors may include a tumor-specific targeting moiety and a labile bond that is selected because it is more likely, more quickly, or more efficiently cleaved in a tumor environment than in the systemic environment. The microenvironment of tumors is often characterized as having low oxygen tension, low extracellular pH, and low glucose concentration. Labile bonds that can exploit one or more of these microenvironmental conditions, e.g., low pH, may make a labile bond particularly well suited for use in a conjugate designed for treating the tumor.

Leuteinizing hormone releasing hormone (LHRH) receptors are significantly elevated on breast cancer, prostate cancer, endometrial cancer, ovarian cancer, and melanoma cells. Thus, ligands of LHRH receptors may be used as a targeting moiety in a conjugate to provide tumor-specific targeted delivery of the IRM moiety to a tumor site. In animal models for the human cancers noted above, LHRH-directed therapeutics selectively home to the affected tissues. Coupling an IRM to a ligand of the LHRH receptor (e.g., LHRH or a synthetic analog) can provide targeted delivery of the IRM to tumor cells of these cancers, thereby concentrating the IRM at the site of the tumor and increasing the therapeutic index over that observed with the IRM compound alone. In studies comparing free Dox to LHRH-conjugated Dox,

approximately 200 times more free Dox was required to demonstrate an antitumor activity equal to the LHRH conjugate. Suitable LHRH receptor ligands could include LHRH decapeptide, an analog with agonist or antagonist activity, or a small molecule receptor ligand.

LHRH receptor is known to be overexpressed on many tumor cells (e.g., breast, prostate, melanoma) compared to normal organ tissues. Thus, a single IRM-LHRH receptor ligand conjugate could be useful for treating more than one type of cancer.

Folic acid receptor ligands also may be useful as targeting moieties that provide tumor-specific targeted delivery of the IRM moiety. The expression of folic acid receptors is increased on the surface of many tumor cells. Once again, coupling a folic acid receptor ligand to an IRM moiety can result in selective accumulation of the IRM at a tumor site, reducing systemic availability of the IRM moiety, and increasing the therapeutic index of the IRM moiety. Suitable folic acid receptor ligands include folic acid, an analog with agonist or antagonist activity, or a small molecule receptor ligand.

In some alternative embodiments, an IRM moiety may be conjugated to a dendritic cell targeting moiety. The targeting moiety may be an antibody (e.g., an anti-DC antibody) or a non-antibody ligand that recognizes a DC-specific marker.

Suitable DC-specific markers may include, for example, a co-stimulatory marker such as, for example, any member of the TNFR Superfamily (e.g., CD40), CD70, CD80, CD86, B7-CD, B7.1, B7.2, etc. A conjugate that includes a targeting moiety that recognizes a co-stimulatory marker may be used to deliver two DC-activating stimuli (i.e., IRM moiety and co-stimulation) in a single chemical entity.

As used herein, an anti-DC antibody refers to an antibody that recognizes a dendritic cell antigen. A suitable dendritic cell targeting moiety may bind to any antigen that is differentially expressed, either qualitatively or quantitatively, by dendritic cells. Suitable dendritic cell targeting moieties may bind to such antigens as, for example, DEC205, BDCA-1, BDCA-2, BDCA-3, BDCA-4, DC-SIGN, L-SIGN, HLR-DR, CD11c, CD13, CD14, CD21, CD33, CD35, CD123, C-type lectins, integrins (e.g., $\alpha 4$, $\alpha 6$, $\alpha 1\beta 1$), and/or any one of the Toll-like receptors (TLRs), etc.

Regardless of whether the targeting moiety recognized a DC-specific marker or antigen, conjugating the IRM moiety to the targeting moiety can limit systemic availability of the IRM moiety, even when administered via a systemic delivery route. Moreover, the conjugate, and thus the IRM moiety, may be concentrated in the vicinity of dendritic cells, thereby maturing and activating dendritic cells more effectively. Dendritic cells activated at the site of a tumor-or even inside a tumor mass-may be able to utilize a tumor antigen present on the surface of the tumor cells to initiate an immune response against the tumor. This method could provide a generalized anti-tumor therapy without the need for tumor-specific antibodies.

In other alternative embodiments, an IRM moiety may be conjugated to an anti-macrophage targeting moiety. Macrophages are often localized in the vicinity of tumor cells. Thus, again, systemic availability of the IRM moiety can be limited, and the IRM moiety may be concentrated in the vicinity of the target cells (i.e., macrophages), thereby activating macrophages more efficiently. Activated macrophages are known to possess anti-tumor activity. Thus, this method could provide a generalized tumor therapy without the need for tumor-specific antibodies.

In other alternative embodiments, an IRM moiety may be conjugated to a target specific moiety that recognizes a surface antigen on a cell type that can directly kill tumor cells such as, for example, $CD8^+$ cytotoxic T cells, NK cells, or NKT cells. Once again, even if the conjugate is administered systemically, the IRM moiety may be concentrated in the vicinity of the tumor-killing cells, thereby (a) activating tumor-killing cells more effectively, and/or (b) limiting the systemic availability of the IRM moiety. Tumor-killing cells activated at the site of a tumor-or even inside a tumor mass-may be able to utilize a tumor antigen present on the surface of the tumor cells to initiate an immune response against the tumor. This method could provide a generalized tumor therapy without the need for tumor-specific antibodies.

In other alternative embodiments, the IRM moiety may be conjugated to a targeting moiety that recognizes, for example, an endothelial target. Significant differences exist in the endothelium environments of tumor masses compared to normal capillary beds. Differences exist, for example, in the identity and extent to which certain endothelial surface proteins, adhesion molecules (e.g., integrins), extracellular matrix proteins, growth factor receptors, etc. are expressed. These differences can be exploited to target delivery of an IRM moiety to tumor-related endothelium. Some reagents that specifically target such differences have been demonstrated to be useful as anti-angiogenic therapies. Conjugating such an agent, as a targeting moiety, to an IRM moiety can combine two effective anti-tumor therapies: immunotherapy and anti-angiogenesis therapy.

Suitable anti-angiogenesis reagents include, for example, anti-CD105 antibodies (CD105 is overexpressed in tumor endothelium), anti-ED-B antibodies (ED-B is a fibronectin isoform found in tumor masses), peptides recognized by endothelial integrins associated with tumors, and growth factors whose receptors are upregulated on tumor endothelium (e.g., vascular endothelial growth factor).

The use of anti-angiogenic reagents in this way may offer the promise of combined anti-angiogenesis and immunotherapy. Additionally, targeted delivery of an IRM to the tumor endothelium, as opposed to the tumor itself, may provide more effective long-term treatment since, generally, the endothelium is a less mutagenic tissue than a tumor mass. Therefore, therapy directed toward the endothelium may be far less likely to cause drug resistance. Also, a therapy

directed toward the endothelium may be effective against virtually any vascularized tumor (e.g., breast cancer, prostate cancer, lung cancer) without the need for tumor-specific reagents.

In some embodiments, a targeting moiety may include an immunoglobulin or at least a functional portion of an immunoglobulin. Because immunoglobulins are proteins, it is understood that modifications can be made to a particular immunoglobulin without rendering the modified immunoglobulin unsuitable for use as a targeting moiety. For example, one or more portions of the immunoglobulin amino acid sequence may be deleted or substituted, or additional amino acids may be added to an immunoglobulin, and the immunoglobulin can still retain sufficient immunospecific character to be suitable for use as a targeting moiety. Examples of suitable antibodies are described, for example, in U.S. Patent Publication No. 2006/0142202 (Alkan et al.).

## Pharmaceutical Compositions and Biological Activity

Pharmaceutical compositions of the disclosure are also contemplated. Pharmaceutical compositions of the disclosure contain a therapeutically effective amount of a compound or salt or conjugate (i.e., complex) of the disclosure (described herein) in combination with a pharmaceutically acceptable carrier.

The compounds of Formula (I) or IRM-containing conjugates of Formula (V-A), or combinations thereof, may be provided in any pharmaceutical composition suitable for administration to a subject (human or animal) and may be present in the pharmaceutical composition in any suitable form (for example as a solution, a suspension, an emulsion, or any form of a mixture). The pharmaceutical composition may be formulated with any pharmaceutically acceptable carrier (e.g., excipient or vehicle). In some embodiments, the pharmaceutically acceptable carrier comprises water (for example phosphate buffered saline or citrate buffered saline). In some embodiments, the pharmaceutically carrier comprises an oil (for example corn, sesame, cottonseed, soybean, or safflower oil). The pharmaceutical composition may further include one or more additives including suspending agents, surfactants, dispersing agents, and preservatives (such as an anti-oxidant).

In some embodiments of the pharmaceutical composition, the compounds of Formula (I) or IRM-containing conjugates of Formula (V-A), or combinations thereof, can be incorporated in a homogeneously dispersed formulation. In some embodiments of the pharmaceutical composition, the compounds of Formula (I) or IRM-containing conjugates of Formula (V-A), or combinations thereof, can be incorporated in an emulsified formulation. In some embodiments of the pharmaceutical composition, the compounds of Formula (I) or IRM-containing conjugates of Formula (V-A), or combinations thereof, can be incorporated in an oil-in-water formulation. An oil-in-water formulation can comprise an oil component, an aqueous component, and one or more surfactants (for example formulations comprising soybean oil, TWEEN 80, SPAN 85, and phosphate buffered saline). In some embodiments of the pharmaceutical composition, the compounds of Formula (I) or IRM-containing conjugates of Formula (V-A), or combinations thereof, can be incorporated into a liposome formulation.

In some embodiments, the pharmaceutical composition can further comprise an antigen in an amount effective to generate an immune response against the antigen. In some embodiments, the antigen is a vaccine.

The pharmaceutical composition can be administered in any suitable manner (parenterally or non-parenterally). In some embodiments, the pharmaceutical composition can be administered by an intradermal, subcutaneous, intramuscular, or intravenous injection.

The exact amount of compound, salt, or IRM-containing conjugate used in a pharmaceutical composition of the disclosure will vary according to factors known to those of skill in the art, such as the physical and chemical nature of the compound, salt, or IRM-containing conjugate, the nature of the carrier, and the intended dosing regimen.

In some embodiments, the concentration of a compound of Formula (I) or IRM-containing conjugates of Formula (V-A), or combinations thereof, in the pharmaceutical composition can be at least 0.0005 mg/mL, at least 0.001 mg/mL, or at least 0.05 mg/mL. In some embodiments, the concentration of a compound of Formula (I) or IRM-containing conjugates of Formula (V-A), or combinations thereof, in the pharmaceutical composition can be up to 2.4 mg/mL, up to 0.06 mg/mL, up to 0.01 mg/mL, or up to 0.005 mg/mL.

In some embodiments, the compositions of the disclosure will contain sufficient active ingredient or prodrug to provide a dose of at least 100 nanograms per kilogram (ng/kg), or at least 10 micrograms per kilogram ($\mu$g/kg), of the compound, salt, or conjugate to the subject. In some embodiments, the compositions of the disclosure will contain sufficient active ingredient or prodrug to provide a dose of up to 50 milligrams per kilogram (mg/kg), or up to 5 mg/kg, of the compound, salt, or conjugate to the subject.

In some embodiments, the compositions of the disclosure will contain sufficient active ingredient or prodrug to provide a dose of, for example, from 0.01 mg/$m^2$ to 5.0 mg/$m^2$, computed according to the Dubois method, in which the body surface area of a subject ($m^2$) is computed using the subject's body weight: $m^2$ = (wt $kg^{0.425}$ x height $cm^{0.725}$) x 0.007184, although in some embodiments the methods may be performed by administering a compound, salt, or conjugate in a dose outside this range. In some of these embodiments, the method includes administering sufficient compound, salt, or conjugate to provide a dose of from 0.1 mg/$m^2$ to 2.0 mg/$m^2$ to the subject, for example, a dose of from

0.4 mg/m$^2$ to 1.2 mg/m$^2$.

In any embodiment of a pharmaceutical composition comprising a compound of Formula (I), the compound of Formula (I) is present in the composition in at least 80% enantiomeric excess, at least 90% enantiomeric excess, at least 95% enantiomeric excess, at least 96% enantiomeric excess, at least 96% enantiomeric excess, at least 97% enantiomeric excess, at least 98% enantiomeric excess, at least 99% enantiomeric excess, at least 99.5% enantiomeric, or at least 99.8% enantiomeric excess.

In any embodiment of a pharmaceutical composition comprising a compound of Formula (I), the opposite enantiomer to the compound is present in the composition in less than 10%, less than 5%, less than 2.5%, less than 2%, less than 1.5%, less than 1%, less than 0.5%, less than 0.25%, or less than 0.1%.

A variety of dosage forms may be used to administer the compounds, salts, or conjugates of the disclosure to a human or animal. Dosage forms that can be used include, for example, tablets, lozenges, capsules, parenteral formulations, creams, ointments, topical gels, aerosol formulations, liquid formulations (e.g., aqueous formulation), transdermal patches, and the like. These dosage forms can be prepared with conventional pharmaceutically acceptable carriers and additives using conventional methods, which generally include the step of bringing the active ingredient into association with the carrier. A preferred dosage form has one or more of compounds, salts, or conjugates of the disclosure dissolved in an aqueous formulation.

Compounds, salts, or conjugates disclosed herein induce the production of certain cytokines in experiments performed according to the description of the Examples. These results indicate that the compounds, salts, or conjugates are useful for enhancing the immune response in a number of different ways, making them useful in the treatment of a variety of disorders.

The compounds, salts, or conjugates described herein can be administered as the single therapeutic agent in the treatment regimen, or the compounds, salts, or conjugates described herein may be administered in combination with other active agents, including antivirals, antibiotics, proteins, peptides, oligonucleotides, antibodies, etc.

Compounds, salts, or conjugates described herein induce the production of cytokines (e.g., IFN-alpha, IFN-gamma, TNF-alpha, IP-10). These results indicate that the compounds, salts, or conjugates of the disclosure are useful for activating the immune response in a number of different ways, rendering them useful in the treatment of a variety of disorders. As such, the compounds, salts, or conjugates of the disclosure are agonists of cytokine biosynthesis and production, particularly agonists of IFN-alpha, IFN-gamma, TNF-alpha, and IP-10 cytokine biosynthesis and production.

It is believed that one way in which the compounds, salts, or conjugates of the disclosure induce cytokine production is through the activation of Toll-like receptors (TLRs) in the immune system, particularly TLR-7 and/or TLR-8, however other mechanisms may be involved. It is believed that in the immune system pathways (i.e., mechanisms) for cytokine induction, the compounds, salts, or conjugates of the disclosure primarily act as agonists of TLR-7 and/or TLR-8, however, other pathways or activities may be involved.

Administration of the compounds, salts, or conjugates described herein can induce the production of interferon-alpha (IFN-alpha), interferon-gamma (IFN-gamma), tumor necrosis factor-alpha (TNF-alpha), and IP-10 in cells. Cytokines whose biosynthesis can be induced by compounds, salts, or conjugates of the disclosure include IFN-alpha, IFN-gamma, TNF-alpha, IP-10, and a variety of other cytokines. Among other effects, these cytokines can inhibit virus production and tumor cell growth, making the compounds or salts useful in the treatment of viral diseases and neoplastic diseases. Accordingly, the disclosure provides a method of inducing cytokine biosynthesis in a human or animal by administering an effective amount of a compound, salt, or conjugate of the disclosure to the human or animal. The human or animal to which the compound, salt, or conjugate is administered for induction of cytokine production may have one or more diseases, disorders, or conditions described below, for example a viral disease or a neoplastic disease, and administration of the compound, salt, or conjugate may provide therapeutic treatment. Alternatively, the compound, salt, or conjugate may be administered to the human or animal prior to the human or animal acquiring the disease so that administration of the compound, salt, or conjugate may provide a prophylactic treatment.

**In** addition to the ability to induce the production of cytokines, compounds, salts, or conjugates described herein can affect other aspects of the innate immune response. For example, natural killer cell activity may be stimulated, an effect that may be due to cytokine induction. The compounds, salts, or conjugates may also activate macrophages, which in turn stimulate secretion of nitric oxide and the production of additional cytokines. **In** addition, the compounds, salts, or conjugates may cause proliferation and differentiation of B-lymphocytes.

Conditions for which compounds, salts, conjugates, or compositions identified herein may be used as treatment include, but are not limited to:

Viral diseases such as, for example, diseases resulting from infection by an adenovirus, a herpes virus (e.g., HSV-I, HSV-II, CMV, or VZV), a poxvirus (e.g., an orthopoxvirus such as variola or vaccinia, or molluscum contagiosum), a picornavirus (e.g., rhinovirus or enterovirus), an orthomyxovirus (e.g., influenza virus, avian influenza), a paramyxovirus (e.g., parainfluenza virus, mumps virus, measles virus, and respiratory syncytial virus (RSV), a coronavirus (e.g., SARS), a papovavirus (e.g., papillomaviruses, such as those that cause genital warts, common warts, or plantar

warts), hepadnavirus (e.g., hepatitis B virus), a flavivirus (e.g., hepatitis C virus or Dengue virus), or a retrovirus (e.g., a lentivirus such as HIV), ebola virus;

Neoplastic diseases such as bladder cancer, cervical dysplasia, cervical cancer, actinic keratosis, basal cell carcinoma, cutaneous T-cell lymphoma, mycosis fungoides, Sezary Syndrome, HPV associated head and neck cancer (e.g., HPV positive oropharyngeal squamous cell carcinoma), Kaposi's sarcoma, melanoma, squamous cell carcinoma, renal cell carcinoma, acute myeloid leukemia, chronic myeloid leukemia, chronic lymphocytic leukemia, multiple myeloma, Hodgkin's lymphoma, non-Hodgkin's lymphoma, B-cell lymphoma, hairy cell leukemia, esophageal cancer, and other cancers;

$T_H2$-mediated atopic diseases such as atopic dermatitis or eczema, eosinophilia, asthma, allergy, allergic rhinitis, and Omenn's syndrome;

Diseases associated with wound repair, such as, for example, inhibition of keloid formation and other types of scarring (e.g., enhancing wound healing, including chronic wounds); and

Parasitic diseases including but not limited to malaria, leishmaniasis, cryptosporidiosis, toxoplasmosis, and trypanosome infection.

In addition, a compound, salt, conjugate, or pharmaceutical composition described herein may be used as a vaccine adjuvant for use in conjunction with any material that increases either humoral and/or cell mediated immune responses, such as, for example, tumor antigens (e.g., MAGE-3, NY-ESO-1); live viral, bacterial, or parasitic immunogens; inactivated viral, protozoal, fungal, or bacterial immunogens; toxoids; toxins; polysaccharides; proteins; glycoproteins; peptides; cellular vaccines; DNA vaccines; autologous vaccines; recombinant proteins; and the like.

Examples of vaccines that can benefit from use of a compound, salt, conjugate, or composition identified herein as a vaccine adjuvant include BCG vaccine, cholera vaccine, plague vaccine, typhoid vaccine, hepatitis A vaccine, hepatitis B vaccine, hepatitis C vaccine, influenza A vaccine, influenza B vaccine, malaria vaccine, parainfluenza vaccine, polio vaccine, rabies vaccine, measles vaccine, mumps vaccine, rubella vaccine, yellow fever vaccine, tetanus vaccine, diphtheria vaccine, hemophilus influenza b vaccine, tuberculosis vaccine, meningococcal and pneumococcal vaccines, adenovirus vaccine, coronavirus vaccine, HIV vaccine, chicken pox vaccine, cytomegalovirus vaccine, dengue vaccine, feline leukemia vaccine, fowl plague vaccine, HSV-1 vaccine and HSV-2 vaccine, hog cholera vaccine, Japanese encephalitis vaccine, respiratory syncytial virus vaccine, rotavirus vaccine, papilloma virus vaccine, yellow fever vaccine, ebola virus vaccine.

Compounds, salts, conjugates, or pharmaceutical compositions identified herein may be particularly useful as vaccine adjuvants when used in conjunction with tumor antigens associated with colorectal cancer, head and neck cancer, breast cancer, lung cancer, and melanoma.

Compounds, salts, conjugates, or pharmaceutical compositions identified herein may be particularly useful in individuals having compromised immune function. For example, compounds, salts, conjugates, or compositions may be used for treating opportunistic infections and tumors that occur after suppression of cell mediated immunity in, for example, transplant patients, cancer patients, and HIV patients.

One or more of the above diseases or types of diseases, for example, a viral disease or neoplastic disease may be treated in a human or animal in need thereof (having the disease) by administering a therapeutically effective amount of a compound, salt, conjugate, or composition to the human or animal.

A human or animal may also be vaccinated by administering an effective amount of a compound, salt, conjugate, or composition described herein as a vaccine adjuvant. In one embodiment, a method of vaccinating a human or animal includes administering an effective amount of a compound, salt, conjugate, or composition described herein to the human or animal as a vaccine adjuvant. The vaccine adjuvant can be co-administered with the material that increases one or more humoral and cell mediated immune responses by including each in the same composition. Alternatively, the vaccine adjuvant and the material that increases either humoral and/or cell mediated immune responses can be in separate compositions.

Compounds, salts, conjugates, or compositions identified herein may as prophylactic or therapeutic vaccine adjuvants in veterinary applications. Compounds, salts, conjugates, or compositions identified herein may be administered to, for example, pigs, horses, cattle, sheep, dogs, cats, poultry (such as chickens or turkeys), etc.

Compounds, salts, conjugates, or compositions identified herein may be particularly useful when an effective amount is administered to a human or animal to treat bladder cancer, cervical dysplasia, actinic keratosis, basal cell carcinoma, genital warts, herpes virus infection, or cutaneous T-cell lymphoma. For these conditions, administration of the compound, salt, or composition of the disclosure is preferably topical (i.e., applied directly to the surface of a tumor, a lesion, a wart, or an infected tissue, etc.).

In one embodiment an effective amount of compound, salt, conjugate, or composition described herein, such as an aqueous composition is administered into the bladder of a human or animal that has at least one tumor of the bladder by intravesical instillation (e.g., administration using a catheter).

An amount of a compound, salt, or conjugate effective to induce cytokine biosynthesis will typically cause one or

more cell types, such as monocytes, macrophages, dendritic cells, and B-cells to produce an amount of one or more cytokines, such as, for example, IFN-alpha, IFN-gamma, TNF-alpha, and IP-10 that is increased (induced) over a background level of such cytokines. The precise dose will vary according to factors known in the art but is typically to be a dose of 100 ng/kg to 50 mg/kg, or 10 μg/kg to 5 mg/kg. In other embodiments, the amount can be, for example, from 0.01 $mg/m^2$ to 5.0 $mg/m^2$ (computed according to the Dubois method as described above), although in other embodiments the induction of cytokine biosynthesis may be performed by administering a compound or salt in a dose outside this range. In some of these embodiments, the method includes administering sufficient compound, salt, conjugate, or composition to provide a dose from 0.1 $mg/m^2$ to 2.0 $mg/m^2$ to the subject, for example, a dose of from 0.4 $mg/m^2$ to 1.2 $mg/m^2$.

A method of treating a viral infection in a human or animal and a method of treating a neoplastic disease in a human or animal can include administering an effective amount of a compound, salt, or conjugate described herein to the human or animal.

An effective amount to treat or inhibit a viral infection can be an amount that will cause a reduction in one or more of the manifestations of viral infection, such as viral lesions, viral load, rate of virus production, and mortality as compared to untreated humans or animals. The precise amount that is effective for such treatment will vary according to factors known in the art but it is normally a dose of 100 ng/kg to 50 mg/kg, or 10 μg/kg to 5 mg/kg.

An amount of a compound, salt, or conjugate effective to treat a neoplastic condition can be an amount that causes a reduction in tumor size or in the number of tumor foci. The precise amount will vary according to factors known in the art but is typically 100 ng/kg to 50 mg/kg, or 10 μg/kg to 5 mg/kg. In other embodiments, the amount is typically, for example, from 0.01 $mg/m^2$ to 5.0 $mg/m^2$ (computed according to the Dubois method as described above), although in some embodiments the induction of cytokine biosynthesis may be performed by administering a compound, salt, or conjugate in a dose outside this range. In some of these embodiments, the method includes administering sufficient compound, salt, conjugate, or composition to provide a dose from 0.1 $mg/m^2$ to 2.0 $mg/m^2$ to the subject, for example, a dose of from 0.4 $mg/m^2$ to 1.2 $mg/m^2$.

## EXEMPLARY EMBODIMENTS

Embodiment 1 is a compound of Formula (I), or a salt thereof:

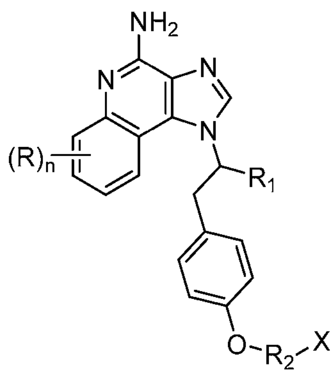

wherein: n is an integer of 0 or 1; R is selected from the group consisting of halogen, hydroxy, alkyl, alkoxy, and -C(O)-O-alkyl; $R_1$ is -$C_{1-3}$alkylene-O-$C_{1-3}$alkyl, $R_2$ is a $C_{2-18}$alkylene group or $C_{2-18}$alkenylene group, optionally interrupted by one or more non-peroxidic -O- atoms; and X is $NH_2$.

Embodiment 4 is the compound or salt of any of the previous embodiments, wherein R is selected from the group consisting of halogen, hydroxy, alkyl (preferably, -$C_{1-7}$alkyl), and alkoxy (preferably, -$C_{1-7}$alkoxy). Embodiment 5 is the compound or salt of embodiment 4, wherein R is selected from the group consisting of hydroxy, F, and Cl. Embodiment 6 is the compound or salt of embodiment 5, wherein R is selected from the group consisting of F and Cl. Embodiment 7 is the compound or salt of any of the previous embodiments, wherein n is 0. Embodiment 8 is the compound or salt of any of the previous embodiments, wherein $R_1$ is -$CH_2OCH_3$ or -$CH_2OCH_2CH_3$. Embodiment 9 is the compound or salt of embodiment 8, wherein $R_1$ is -$CH_2OCH_2CH_3$. Embodiment 10 is the compound or salt of any of the previous embodiments, wherein $R_2$ is a $C_{2-18}$alkylene group optionally interrupted by one or more non-peroxidic -O- atoms. Embodiment 11 is the compound or salt of embodiment 10, wherein $R_2$ is a $C_{2-12}$ alkylene group. Embodiment 12 is the compound or salt of embodiment 11, wherein $R_2$ is a $C_{2-6}$alkylene group. Embodiment 13 is the compound or salt of embodiment 12, wherein $R_2$ is a $C_{2-4}$alkylene group.

Embodiment 14 is a compound of Formula (II), or a salt thereof:

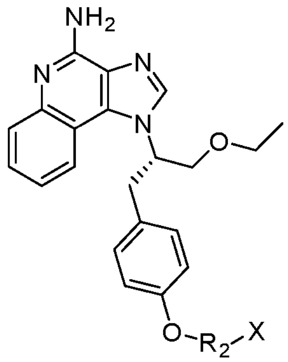

wherein: $R_2$ is a $C_{2-18}$alkylene group or $C_{2-18}$alkenylene group, optionally interrupted by one or more non-peroxidic -O- atoms; and X is $NH_2$.

Embodiment 15 is the compound or salt of embodiment 14, wherein $R_2$ is a $C_{2-18}$alkylene group optionally interrupted by one or more non-peroxidic -O- atoms. Embodiment 16 is the compound or salt of embodiment 15, wherein $R_2$ is a $C_{2-12}$ alkylene group. Embodiment 17 is the compound or salt of embodiment 16, wherein $R_2$ is a $C_{2-6}$alkylene group. Embodiment 18 is the compound or salt of embodiment 17, wherein $R_2$ is a $C_{2-4}$alkylene group.

Reference Embodiment 19 is a compound of Formula (III), or salt thereof:

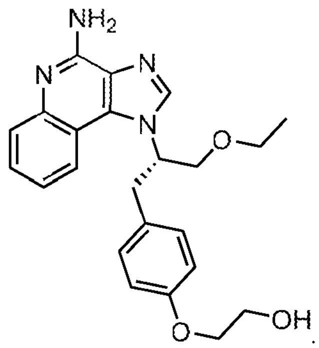

Embodiment 20 is a compound of Formula (IV), or salt thereof:

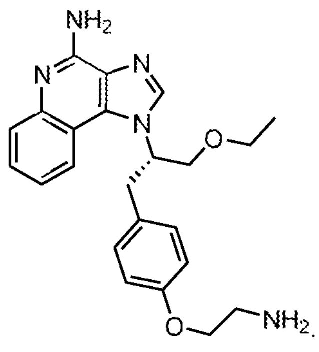

Embodiment 21 is an IRM-containing conjugate of Formula (V-A):

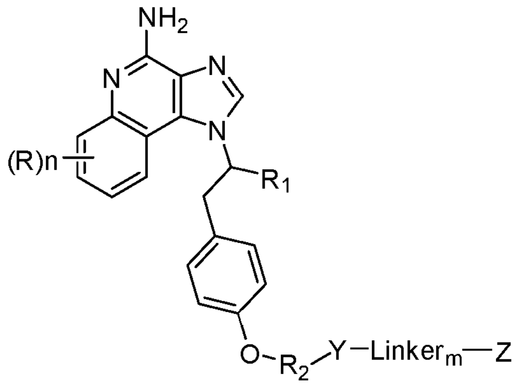

wherein: n is an integer of 0 or 1; R is selected from the group consisting of halogen, hydroxy, alkyl, alkoxy, and -C(O)-O-alkyl; $R_1$ is $-C_{1-3}$alkylene-O-$C_{1-3}$alkyl ; $R_2$ is a $C_{2-18}$alkylene group or $C_{2-18}$alkenylene group, optionally interrupted by one or more non-peroxidic -O- atoms; Y is N or NH; Linker is a heterobifunctional crosslinking group as outlined in claim 8; m = 0 or 1; Z is an antibody.

Embodiment 23 is the conjugate of embodiment 21, wherein Y is NH. Embodiment 24 is the conjugate of embodiment 21, wherein Y is N. Embodiment 25 is the conjugate of any of embodiments 21 through 24, wherein R is selected from the group consisting of halogen, hydroxy, alkyl (preferably, $-C_1$-$_7$alkyl), and alkoxy (preferably, $-C_1$-$_7$alkoxy). Embodiment 26 is the conjugate of embodiment 25, wherein R is selected from the group consisting of hydroxy, F, and Cl. Embodiment 27 is the conjugate of embodiment 26, wherein R is selected from the group consisting of F and Cl. Embodiment 28 is the conjugate of any of embodiments 21 through 27, wherein n is 0. Embodiment 29 is the conjugate of any of embodiments 21 through 28, wherein $R_1$ is - $CH_2OCH_3$ or $-CH_2OCH_2CH_3$. Embodiment 30 is the conjugate of embodiment 29, wherein $R_1$ is - $CH_2OCH_2CH_3$. Embodiment 31 is the conjugate of any of embodiments 21 through 30, wherein $R_2$ is a $C_{2-18}$alkylene group optionally interrupted by one or more non-peroxidic -O- atoms. Embodiment 32 is the conjugate of embodiment 31, wherein $R_2$ is a $C_{2-12}$alkylene group. Embodiment 33 is the conjugate of embodiment 32, wherein $R_2$ is a $C_{2-6}$alkylene group. Embodiment 34 is the conjugate of embodiment 33, wherein $R_2$ is a $C_{2-4}$alkylene group.

Embodiment 40 is an IRM-containing conjugate of Formula (V-B):

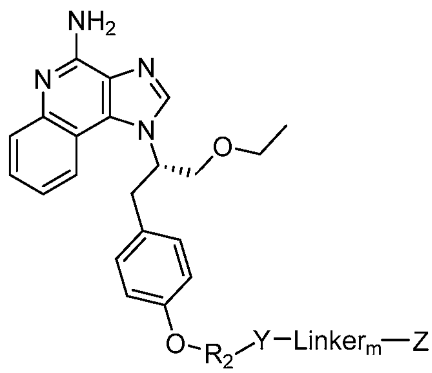

wherein: $R_2$ is a $C_{2-18}$alkylene group or $C_{2-18}$alkenylene group, optionally interrupted by one or more non-peroxidic -O-atoms; Y is N or NH; Linker is a heterobifunctional crosslinking group as outlined in claim 8; m = 0 or 1; Z is an antibody.

Embodiment 42 is the conjugate of embodiment 40, wherein Y is NH. Embodiment 43 is the conjugate of embodiment 40, wherein Y is N. Embodiment 44 is the conjugate of embodiment 40, wherein $R_2$ is a $C_{2-18}$alkylene group optionally interrupted by one or more non-peroxidic -O- atoms. Embodiment 45 is the conjugate of embodiment 44, wherein $R_2$ is a $C_{2-12}$alkylene group. Embodiment 46 is the conjugate of embodiment 45, wherein $R_2$ is a $C_{2-6}$alkylene group. Embodiment 47 is the conjugate of embodiment 46, wherein $R_2$ is a $C_{2-4}$alkylene group.

Embodiment 53 is a pharmaceutical composition comprising the compound of any of embodiments 1 through 18 or 20 and a pharmaceutically acceptable carrier. Embodiment 54 is a pharmaceutical composition comprising the IRM-containing conjugate of any of embodiments 21 through 46 and a pharmaceutically acceptable carrier.

Embodiment 55 is the pharmaceutical composition of embodiment 53 or 54 for use in inducing cytokine biosynthesis in a human or animal.

Embodiment 56 is a compound for use in inducing biosynthesis of IFN-alpha in a human or animal comprising administering an effective amount of a pharmaceutical composition of embodiment 53 or 54 to the human or animal.

Embodiment 57 is a compound for use in inducing biosynthesis of IFN-gamma in a human or animal comprising administering an effective amount of a pharmaceutical composition of embodiment 53 or 54 to the human or animal.
Embodiment 58 is a compound for use in inducing biosynthesis of TNF-alpha in a human or animal comprising administering an effective amount of a pharmaceutical composition of embodiment 53 or 54 to the human or animal.
Embodiment 59 is a compound for use in inducing biosynthesis of IP-10 in a human or animal comprising administering an effective amount of a pharmaceutical composition of embodiment 53 or 54 to the human or animal.

EXAMPLES

Objects and advantages of the disclosure are further illustrated by the examples provided herein. The particular materials and amounts thereof recited in these examples, as well as other conditions and details, are merely illustrative and are not intended to be limiting. The person of ordinary skill in the art, after carefully reviewing the entirety of this disclosure, will be able to use materials and conditions in addition to those specifically described in the examples.

Column chromatography purification of compounds was conducted using an ISOLARA HPFC system (an automated high-performance flash chromatography purification instrument available from Biotage, Inc, Charlottesville, VA). The eluent used for each purification is described in the examples.

Proton nuclear magnetic resonance ($^1H$ NMR) analysis was conducted using a BRUKER A500 NMR spectrometer (Bruker Corporation, Billerica, MA).

Cesium carbonate ($Cs_2CO_3$) and benzenesulfonyl chloride were obtained from the Sigma-Aldrich Company, St. Louis, MO.

Triethyl orthoformate, 3% platinum on carbon, n-propyl acetate, 2-(Boc-amino)ethyl bromide and pyridine hydrochloride were obtained from the Alfa Aesar Company, Haverhill, MA.

2-Bromoethyl acetate and 3-chloroperbenzoic acid (about 70% MCPBA, which was determined iodometrically according to Braun, G. Org. Synth., Collective Volume 1932, 1, 431) were obtained from Oakwood Products Incorporated, Estill, SC.

5M sodium methoxide solution in methanol was obtained from TCI America, Portland, OR.

Example 1 (Reference)

(S)-2-(4-(2-(4-amino-1H-imidazo[4,5-c]quinolin-1-yl)-3-ethoxypropyl)phenoxy)ethan-1-ol

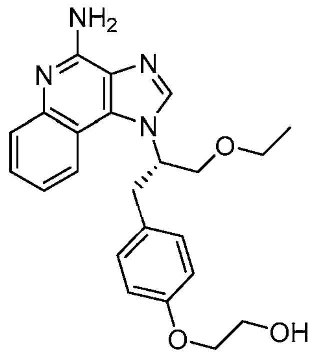

Part A

To a stirred solution of 4-[(2S)-3-ethoxy-2-[(3-nitro-4-quinolyl)amino]propyl]phenol (preparation described in patent WO 2019/166937 (3M)) (2.54 grams (g), 6.92 millimoles (mmol)) dissolved in 25 milliliters (mL) of anhydrous DMF were added $Cs_2CO_3$ (3.37 g, 10.4 mmol) followed by 2-bromoethyl acetate (1.31 g, 7.84 mmol). The reaction mixture was heated to 65 °C under an atmosphere of $N_2$. After 2 hours (h), the reaction mixture was diluted with 75 mL of ethyl acetate and washed with 75 mL of water. The aqueous portion was extracted with an additional 50 mL of ethyl acetate. The combined organic portions were washed with water (5 x 25 mL) and brine, dried over $Na_2SO_4$, filtered and concentrated. Purification by column chromatography ($SiO_2$, 1% $MeOH/CHCl_3$-2% $MeOH/CHCl_3$) gave 2.84 g of (S)-2-(4-(3-ethoxy-2-((3-nitroquinolin-4-yl)amino)propyl)phenoxy)ethyl acetate as a yellow solid.

Part B

A solution of (S)-2-(4-(3-ethoxy-2-((3-nitroquinolin-4-yl)amino)propyl)phenoxy)ethyl acetate (2.80 g, 6.18 mmol) dissolved in 75 mL of acetonitrile was placed in a pressure bottle and combined with 100 milligrams (mg) of 3% Pt on carbon. The bottle was then shaken under an atmosphere of $H_2$ (48 PSI) for 5 h. The reaction mixture was filtered through a pad of CELITE, rinsing with acetonitrile, and the filtrate was concentrated under reduced pressure to give 2.60 g of (S)-2-(4-(2-((3-aminoquinolin-4-yl)amino)-3-ethoxypropyl)phenoxy)ethyl acetate as an orange syrup.

Part C

To a solution of (S)-2-(4-(2-((3-aminoquinolin-4-yl)amino)-3-ethoxypropyl)phenoxy)ethyl acetate (2.60 g, 6.15 mmol) dissolved in 50 mL of n-propyl acetate was added triethyl orthoformate (2.04 mL, 12.3 mmol) and 100 mg of pyridine hydrochloride and the mixture was heated to 105 °C overnight. The cooled reaction mixture was diluted with 50 mL of ethyl acetate and washed successively with saturated $NaHCO_3$ solution, water and brine. The organic portion was dried over $Na_2SO_4$, filtered and concentrated to give an amber syrup. Purification by column chromatography ($SiO_2$, 1% MeOH/$CHCl_3$-10% MeOH/$CHCl_3$) gave 2.72 g of (S)-2-(4-(3-ethoxy-2-(1H-imidazo[4,5-c]quinolin-1-yl)propyl)phenoxy)ethyl acetate as a honey colored syrup.

Part D

A solution of (S)-2-(4-(3-ethoxy-2-(1H-imidazo[4,5-c]quinolin-1-yl)propyl)phenoxy)ethyl acetate (2.72 g, 6.28 mmol) dissolved in 50 mL of $CH_2Cl_2$ was combined with 1.65 mg of MCPBA (70%) and stirred for 2 h. The reaction mixture was cooled to -10 °C followed by addition of 15 mL of concentrated $NH_4OH$ solution. The mixture was stirred rapidly and benzenesulfonyl chloride (0.96 mL, 7.54 mmol) was added dropwise over 1 minute (min). The reaction mixture was then allowed to warm to ambient temperature over 45 min. The reaction was quenched by addition of 20 mL of water and the mixture was stirred for 15 min. The layers were then separated and the organic portion was washed successively with $H_2O$, 5% $Na_2CO_3$ solution and brine. The organic portion was dried over $Na_2SO_4$, filtered and concentrated under reduced pressure. Purification by column chromatography ($SiO_2$, 4% MeOH/$CHCl_3$ saturated with $NH_4OH$) gave 2.12 g of (S)-2-(4-(2-(4-amino-1H-imidazo[4,5-c]quinolin-1-yl)-3-ethoxypropyl)phenoxy)ethyl acetate as an amber foam.

Part E

A solution of (S)-2-(4-(2-(4-amino-1H-imidazo[4,5-c]quinolin-1-yl)-3ethoxypropyl)phenoxy)ethyl acetate (2.10 g) dissolved in 25 mL of anhydrous methanol was combined with 2 drops of 5M sodium methoxide solution. After stirring for 30 min, an additional 3 drops of 5M sodium methoxide solution was added to the reaction mixture and stirring was continued for 90 min. The reaction mixture was concentrated and subjected to purification by column chromatography ($SiO_2$, 5% MeOH/$CHCl_3$ saturated with $NH_4OH$) to give 1.10 g of a light brown foam. The foam was dissolved in 20 mL of $CHCl_3$ and washed successively with 1N NaOH (2 x 10 mL), water and brine. The organic portion was dried over $Na_2SO_4$, filtered and concentrated. The resulting material was dissolved in 20 mL of ethanol and 1 mL of concentrated hydrochloric acid and the mixture was concentrated to dryness. The material was then concentrated from ethanol and then from acetonitrile to give a white solid. The white solid was heated in 20 mL of refluxing acetonitrile and the mixture was allowed to cool to ambient temperature overnight. The resulting solid was isolated by filtration, rinsed with acetonitrile to dried under vacuum to give 818 mg of (S)-2-(4-(2-(4-amino-1H-imidazo[4,5-c]quinolin-1-yl)-3-ethoxypropyl)phenoxy)ethan-1-ol hydrochloride as a light yellow solid. $^1H$ NMR (500 MHz, DEUTERIUM OXIDE) $\delta$ 8.26 (s, 1H), 7.82 (d, *J*=8.3 Hz, 1H), 7.43 (d, *J*=7.6 Hz, 1H), 7.35 (d, *J*=8.3 Hz, 1H), 7.25 (t, *J*=7.7 Hz, 1H), 6.48 (d, *J*=7.3 Hz, 2H), 6.29 (d, *J*=7.6 Hz, 2H), 5.31 (m, 1H), 3.94 (d, *J*=6.0 Hz, 2H), 3.62-3.55 (m, 4H), 3.46 (m, 2H), 2.94 (m, 1H), 2.80 (m, 1H), 0.94 (t, *J*=7.0 Hz, 3H).

Example 2

(S)-1-(1-(4-(2-aminoethoxy)phenyl)-3-ethoxypropan-2-yl)-1H-imidazo[4,5-c]quinolin-4-amine

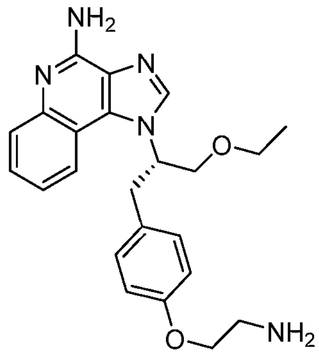

Part A

To a stirred solution of 4-[(2S)-3-ethoxy-2-[(3-nitro-4-quinolyl)amino]propyl]phenol (1.29 g, 3.52 mmol) dissolved in 10 mL of anhydrous DMF were added $Cs_2CO_3$ (1.72 g, 5.28 mmol) followed by 2-(Boc-amino)ethyl bromide (788 mg, 3.52 mmol). The reaction mixture was heated to 65 °C under an atmosphere of $N_2$. After 4 h, another 105 mg of tert-butyl (2-bromoethyl)carbamate was added and the reaction mixture was allowed to cool to ambient temperature overnight. The reaction mixture was then concentrated under reduced pressure and the resulting material was partitioned between 50 mL of ethyl acetate and 50 mL of water. The organic portion was washed successively with water (2 x 20 mL) and brine, dried over $Na_2SO_4$, filtered and concentrated. Purification by column chromatography ($SiO_2$, 1% MeOH/$CHCl_3$-3% MeOH/$CHCl_3$) gave 1.30 g of tert-butyl (S)-(2-(4-(3-ethoxy-2-((3-nitroquinolin-4-yl)amino)propyl)phenoxy)ethyl)carbamate as a yellow syrup.

Part B

A solution of (S)-(2-(4-(3-ethoxy-2-((3-nitroquinolin-4-yl)amino)propyl)phenoxy)ethyl) carbamate (1.30 g, 2.55 mmol) dissolved in 50 mL of acetonitrile was placed in a pressure bottle and combined with 70 mg of 3% Pt on carbon. The bottle was then shaken under an atmosphere of $H_2$ (48 pounds per square inch (PSI)) for 5 h. The reaction mixture was filtered through a pad of CELITE, rinsing with acetonitrile, and the filtrate was concentrated under reduced pressure to give 1.16 g of tert-butyl (S)-(2-(4-(2-((3-aminoquinolin-4-yl)amino)-3-ethoxypropyl)phenoxy)ethyl) carbamate as an orange solid.

Part C

To a solution of tert-butyl (S)-(2-(4-(2-((3-aminoquinolin-4-yl)amino)-3-ethoxypropyl)phenoxy)ethyl)carbamate (1.16 g, 2.42 mmol) dissolved in 50 mL of n-propyl acetate was added triethyl orthoformate (0.80 mL, 4.83 mmol) and 50 mg of pyridine hydrochloride and the mixture was heated to 105 °C overnight. The reaction mixture was then cooled followed by addition of saturated $NaHCO_3$ solution. After stirring for 15 min., the layers were separated and the organic portion was washed successively with water and brine. The organic portion was dried over $Na_2SO_4$, filtered and concentrated to give an amber syrup. Purification by column chromatography ($SiO_2$, 1% MeOH/$CHCl_3$-3% MeOH/$CHCl_3$) gave 1.07 g of tert-butyl (S)-(2-(4-(3-ethoxy-2-(1H-imidazo[4,5-c]quinolin-1-yl)propyl)phenoxy)ethyl)carbamate as an off-white foam.

Part D

A solution of tert-butyl (S)-(2-(4-(3-ethoxy-2-(1H-imidazo[4,5-c]quinolin-1-yl)propyl)phenoxy)ethyl)carbamate (1.07 g, 2.18 mmol) dissolved in 25 mL of $CH_2Cl_2$ was combined with 590 mg of MCPBA (70%) and stirred for 90 min. The reaction mixture was cooled to -10 °C followed by addition of 8 mL of concentrated $NH_4OH$ solution. The mixture was stirred rapidly and benzenesulfonyl chloride (0.34 mL, 2.66 mmol) was added dropwise over 1 min. The reaction mixture was then allowed to warm to ambient temperature over 45 min. The reaction was quenched by addition of 20 mL of water and the mixture was stirred for 15 min. The layers were then separated and the organic portion was washed successively with $H_2O$, 5% $Na_2CO_3$ solution and brine. The organic portion was dried over $Na_2SO_4$, filtered and concentrated under reduced pressure. Purification by column chromatography ($SiO_2$, 4% MeOH/$CHCl_3$ saturated with $NH_4OH$) gave 843 mg of tert-butyl (S)-(2-(4-(2-(4-amino-1H-imidazo[4,5-c]quinolin-1-yl)-3-ethoxypropyl)phenoxy)ethyl)carbamate as an amber foam.

Part E

A solution of tert-butyl (S)-(2-(4-(2-(4-amino-1H-imidazo[4,5-c]quinolin-1-yl)-3-ethoxypropyl)phenoxy)ethyl) carbamate (750 mg, 1.48 mmmol) dissolved in 15 mL of ethanol was combined with 1 mL of concentrated hydrochloric acid and the mixture was heated to reflux for 90 min. The reaction mixture was concentrated to give a syrup. The syrup was then concentrated from ethanol and then from acetonitrile to give a white solid. The white solid was heated in 20 mL of hot acetonitrile with a few drops of ethanol. The hot solution was then transferred to a new flask via pipette leaving behind insoluble material. The solution was cooled to ambient temperature overnight. The resulting solid was isolated by filtration, rinsed with acetonitrile and dried with suction to give 483 mg of (S)-1-(1-(4-(2-aminoethoxy)phenyl)-3-ethoxypropan-2-yl)-1H-imidazo[4,5-c]quinolin-4-amine dihydrochloride as a light tan solid. $^{1}H$ NMR (500 MHz, DEUTERIUM OXIDE) $\delta$ 8.33 (s, 1H), 7.97 (d, *J*=8.3 Hz, 1H), 7.53-7.43 (m, 2H), 7.33 (t, *J*=7.5 Hz, 1H), 6.69 (d, *J*=8.0 Hz, 2H), 6.49 (d, *J*=8.3 Hz, 2H), 5.45 (m, 1H), 4.04-3.97 (m, 2H), 3.89 (t, *J*=4.8 Hz, 2H), 3.52-3.43 (m, 2H), 3.16 (t, *J*=4.7 Hz, 1H), 3.13 (m, 1H), 2.96 (dd, *J*=8.8, 14.1 Hz, 1H), 0.94 (t, *J*=7.0 Hz, 3H).

Cytokine Induction in Human Cells

Whole blood was obtained from healthy human donors and collected by venipuncture into vacutainer tubes or syringes containing EDTA. Human peripheral blood mononuclear cells (PBMC) were purified from the whole blood by density gradient centrifugation. Histopaque 1077 (15 mL, Sigma, St. Louis, MO) was transferred to 6 X 50 mL sterile polypropylene conical tubes. The Histopaque was overlayed with 15-25 mL of blood diluted 1:2 in Hank's Balanced Salts Solution (HBSS) (Gibco, Life Technologies, Grand Island, NY). The tubes were then centrifuged at 1370 revolutions per minute (rpm) for 30 min at 20 °C, with no brake (400Xg, GH 3.8A Rotor).

The interface (buffy coat) containing the PBMC was collected and placed in a new sterile 50 mL conical polypropylene centrifuge tube. The PBMC were mixed with an equal volume of HBSS (about 20 mL from the interface and about 20 mL of HBSS), and then centrifuged at 1090 rpm, 10 min, 20 °C, with brake (270Xg, GH 3.8A Rotor). After completing centrifugation, the cells were resuspended in 2-3 mL ACK Red blood cell lysis buffer (ammonium chloride potassium solution, Gibco, Life Technologies) and incubated for 2-5 min at 20 °C. Next, HBSS (40 mL) was added to the cells, and the sample was centrifuged at 270Xg for 10 min at 20 °C. The supernatant was decanted, and the cell pellet was resuspended in 5 mL AIM V Medium (Gibco, Life Technologies). Cell aggregates and debris were removed by filtering the cell solution through a BD Falcon 70 micron nylon cell strainer (BD Biosciences, San Jose, CA).

The number of viable cells was determined by counting with a Miltenyi FACS instrument (Miltenyi Biotec Inc., San Diego, CA) or by using a hemacytometer. For determining cell viability with a hemacytometer, the cells were diluted 1/10 in 0.4% trypan blue and HBSS (specifically, 50 microliter of trypan blue + 40 microliter of HBSS + 10 microliter of cell solution were added to a microfuge tube and mixed). Ten microliters of the diluted cells were then applied to the hemacytometer, and the number of viable PBMC were determined by microscopy.

The PBMC sample was then resuspended in 96-well plates at a concentration of $8x10^5$ cells/well in 0.1 mL of AIM-V medium. Each compound was solubilized in dimethyl sulfoxide (DMSO) to create a 3 mM stock solution. The stock solution was then further diluted with AIM-V medium to prepare the serial dilutions. The diluted compound (100 microliters) was then transferred to the PBMCs to produce testing sets with final compound concentrations of 30, 10, 3.3, 1.1, 0.37, 0.12, 0.04, 0.01 micromolar. The plates also had both positive and negative controls. The negative control wells contained only AIM-V medium with no example compound. The positive control wells contained a control set of imiquimod serially diluted to concentrations of 30, 10, 3.3, 1.1, 0.37, 0.12, 0.04, 0.01 micromolar. The concentrations used in the control set were selected to match the concentrations used in the testing set. The plates were then cultured at 37 °C /5 % $CO_2$ for 21-24 h. Cell-free supernatants were harvested by centrifuging the 96-well plates at 2100 rpm, 23 °C for 10 min. Approximately 160 microliters of the supernatant was then stored in a NUNC 96-well plate, covered with the compression cap and stored at -80 °C until the cytokine analysis was done.

IFN-alpha cytokine levels (picograms/mL) were measured by ELISA (human IFN-alpha, pan specific, Mabtech, Cincinnati, OH). IFN-gamma and TNF-alpha levels (picograms/mL) were measured by multiplex bead assay (magnetic beads, R & D Systems, Minneapolis, MN) according to the manufacturer's instructions.

The data was analyzed to determine the minimum effective concentration (MEC) for each compound at which induction of a particular cytokine was observed in the assay. Specifically, the minimum effective concentration of each compound (micromolar) was determined as the lowest concentration of the compound that induced a measured cytokine response at a level (pictograms/mL) that was at least 2X greater than that observed with the negative control wells. The results are presented in Table 1. The designation "≤ 0.01" indicate that cytokine induction was observed at the lowest concentration of compound evaluated in the assay.

Table 1. Cytokine Induction

| | MEC to Induce Cytokine (micromolar) | | |
|---|---|---|---|
| Compound | IFN-alpha | IFN-gamma | TNF-alpha |
| Example 1 | ≤ 0.01 | ≤ 0.01 | ≤ 0.01 |
| Example 2 | ≤ 0.01 | ≤ 0.01 | ≤ 0.01 |

TLR Activation and Specificity

HEK-BLUE-hTLR7 or hTLR8 reporter cells were obtained from InvivoGen, San Diego, CA. According to the manufacturer's description, these reporter cells were prepared by co-transfection of HEK293 cells with an inducible secreted embryonic alkaline phosphatase (SEAP) reporter gene and either the human TLR7 or TLR8 gene. The SEAP reporter gene was placed under the control of an IFN-β minimal promoter fused to five NF-κB and AP-1-binding sites. In the presence of a TLR ligand, activation of NF-κB and AP-1 occurs, resulting in a corresponding increase in SEAP levels.

Parental HEK293 cells (null), which expressed the inducible SEAP reporter, but did not express TLR7 or TLR8, were obtained from InvivoGen and served as the negative control in the assay.

In the assay, the HEK cells were grown and maintained using standard cell culture techniques in a growth medium that contained Dulbecco's Modified Eagle Medium (ThermoFisher Scientific Incorporated, Waltham, MA) supplemented with 1% penicillin/streptomycin and 10% heat-inactivated Gibco fetal bovine serum (ThermoFisher Scientific). Each compound was solubilized in DMSO to create a 3 mM stock solution. The stock solution was then further diluted with the growth medium to prepare serial dilutions. Each test compound was tested at a concentration of 30, 10, 3.3, 1.1, 0.37, 0.12, 0.04, and 0.01 micromolar using a 96-well format with $5x10^4$ cells and 200 microliters of growth medium per well.

For each compound, hTLR7, hTLR8, and their respective null control HEK cells were screened. DMSO serially diluted into the growth medium served as the vehicle control. Cell culture supernatants containing the SEAP reporter were collected after an incubation period of 16-20 h in a cell culture incubator (37°C and 5% $CO_2$), and either analyzed immediately or stored at - 80 °C. SEAP levels were measured using the colorimetric enzyme assay (QUANTI-BLUE (InvivoGen) according to manufacturer's instructions.

The data was analyzed to determine the minimum effective concentration (MEC) for each compound at which activation was observed in the assay. Specifically, the minimum effective concentration of each compound (micromolar) was determined as the lowest concentration of the compound that produced a SEAP expression response at least 2X greater than that observed with the vehicle control wells. The results are presented in Table 2. The "designation "≤ 0.01" indicates that TLR activation was observed at the lowest concentration of compound evaluated in the assay.

Table 2. TLR Activation

| | MEC to Produce TLR Activation (micromolar) | |
|---|---|---|
| Compound | TLR 7 | TLR 8 |
| Example 1 | ≤ 0.01 | 1.1 |
| Example 2 | ≤ 0.01 | 1.1 |

Various modifications and alterations to this invention will become apparent to those of ordinary skill in the art without departing from the scope claimed according to this invention. It should be understood that this invention is not intended to be unduly limited by the illustrative embodiments and examples set forth herein and that such examples and embodiments are presented by way of example only with the scope of the invention intended to be limited only by the claims set forth herein as follows.

## Claims

1. A compound of Formula (I), or a salt thereof:

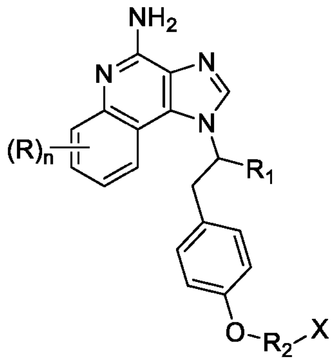

(I)

wherein:

n is an integer of 0 or 1;
R is selected from the group consisting of halogen, hydroxy, alkyl, alkoxy, and -C(O)-O-alkyl;
$R_1$ is -$C_{1-3}$alkylene-O-$C_{1-3}$alkyl;
$R_2$ is a $C_{2-18}$alkylene group or $C_{2-18}$alkenylene group, optionally interrupted by one or more non-peroxidic -O- atoms; and
X is $NH_2$.

**2.** The compound or salt of claim 1, wherein R is selected from the group consisting of halogen, hydroxy, -$C_1$-$_7$alkyl, and -$C_1$-$_7$alkoxy.

**3.** The compound or salt of claim 1, wherein n is 0.

**4.** The compound or salt of any of claims 1 to 3, wherein $R_2$ is a $C_{2-18}$alkylene group optionally interrupted by one or more non-peroxidic -O- atoms.

**5.** The compound or salt of any one of claims 1 to 4, wherein $R_2$ is a $C_{2-6}$ alkylene group.

**6.** The compound or salt of any one of claims 1 to 5 wherein the compound has the Formula (II), or is a salt of the compound of Formula (II):

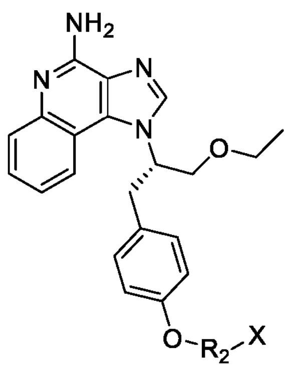

(II).

**7.** The compound or salt of any one of claims 1 to 6, represented by Formula (IV) or a salt thereof:

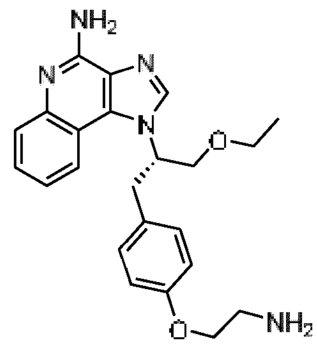

(IV).

**8.** An IRM-containing conjugate of Formula (V-A):

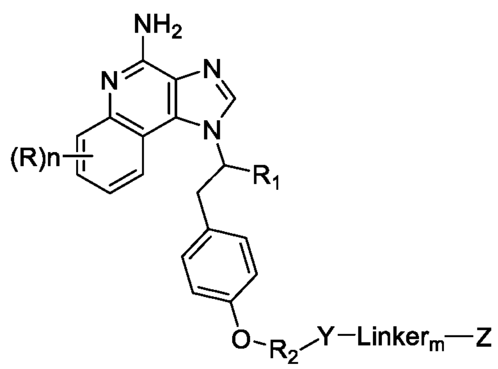

(V-A)

wherein:

n is an integer of 0 or 1;
R is selected from the group consisting of halogen, hydroxy, alkyl, alkoxy, and -C(O)-O-alkyl;
$R_1$ is -$C_{1-3}$alkylene-O-$C_{1-3}$alkyl;
$R_2$ is a $C_{2-18}$alkylene group or $C_{2-18}$alkenylene group, optionally interrupted by one or more non-peroxidic -O- atoms;
Y is N or NH;
Linker is a heterobifunctional crosslinking group derived from:

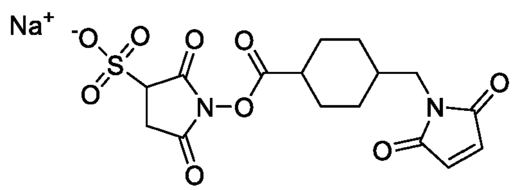

,

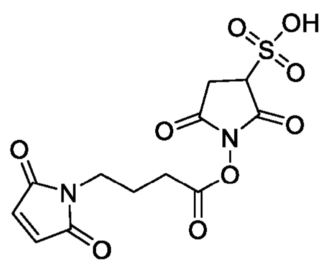

,

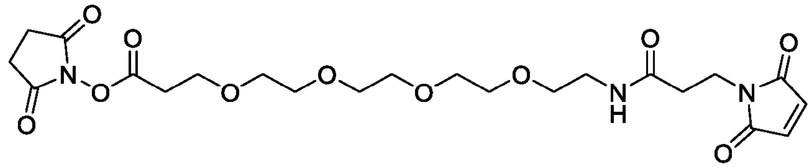

,

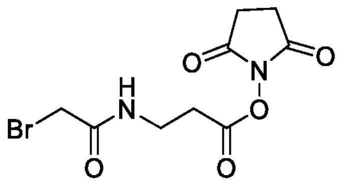
,

or

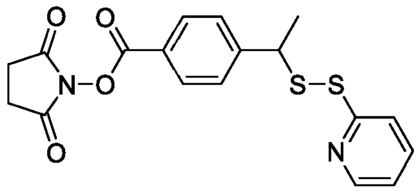
;

m = 0 or 1; and
Z is an antibody.

**9.** The IRM-containing conjugate of claim 8, wherein the IRM-containing conjugate has the Formula (V-B):

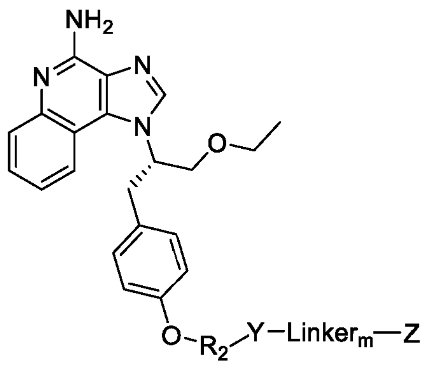

(V-B)

wherein:

Y is NH;
m = 1.

**10.** The IRM-containing conjugate of claim 8 or 9, wherein $R_2$ is a $C_{2-6}$ alkylene group.

**11.** The IRM-containing conjugate of any one of claims 8 to 10, wherein n is 0.

**12.** The IRM-containing conjugate of any one of claims 8 to 11, the Linker derived from:

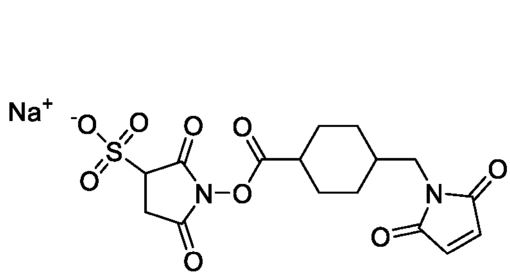
or
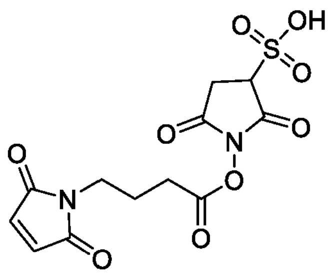
.

**13.** A pharmaceutical composition comprising the compound of any of claims 1 to 8 and a pharmaceutically acceptable carrier.

**14.** A pharmaceutical composition comprising the IRM-containing conjugate of any of claims 9 to 12 and a pharmaceutically acceptable carrier.

**15.** The pharmaceutical composition of claim 13 or 14 for use in inducing cytokine biosynthesis in a human or animal.

## Patentansprüche

**1.** Eine Verbindung der Formel (I) oder ein Salz davon:

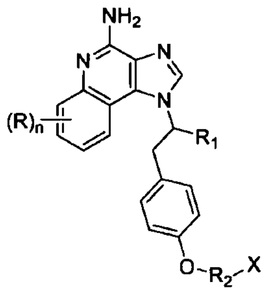

(I)

wobei:

n eine ganze Zahl von 0 oder 1 ist;
R aus der Gruppe ausgewählt ist, bestehend aus Halogen, Hydroxy, Alkyl, Alkoxy und -C(O)-O-Alkyl;
$R_1$ -$C_{1-3}$-Alkylene-O-$C_{1-3}$-Alkyl ist;
$R_2$ eine $C_{2-18}$-Alkylengruppe oder $C_{2-18}$-Alkenylengruppe ist, die gegebenenfalls durch ein oder mehrere nicht-peroxidische -O-Atome unterbrochen ist; und
X $NH_2$ ist.

**2.** Die Verbindung oder das Salz nach Anspruch 1, wobei R aus der Gruppe ausgewählt ist, bestehend aus Halogen, Hydroxy, -$C_{1-7}$-Alkyl und -$C_{1-7}$-Alkoxy.

**3.** Die Verbindung oder das Salz nach Anspruch 1, wobei n 0 ist.

**4.** Die Verbindung oder das Salz nach einem der Ansprüche 1 bis 3, wobei $R_2$ eine $C_{2-18}$-Alkylengruppe ist, die gegebenenfalls durch ein oder mehrere nicht-peroxidische -O-Atome unterbrochen ist.

**5.** Die Verbindung oder das Salz nach einem der Ansprüche 1 bis 4, wobei $R_2$ eine $C_{2-6}$-Alkylengruppe ist.

**6.** Die Verbindung oder das Salz nach einem der Ansprüche 1 bis 5, wobei die Verbindung die Formel (II) aufweist oder ein Salz der Verbindung der Formel (II) ist:

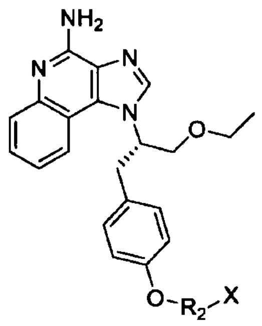

(II).

7. Die Verbindung oder das Salz nach einem der Ansprüche 1 bis 6, die/das durch Formel (IV) oder ein Salz davon dargestellt ist:

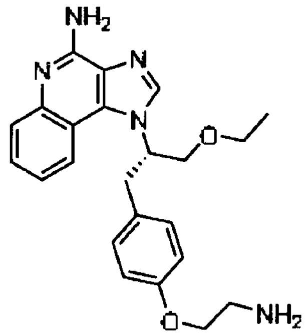

(IV).

8. Ein IRM-enthaltendes Konjugat der Formel (V-A):

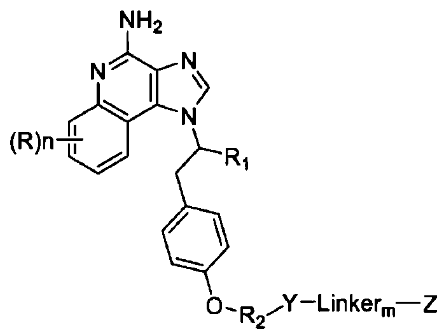

(V-A)

wobei:

n eine ganze Zahl von 0 oder 1 ist;
R aus der Gruppe ausgewählt ist, bestehend aus Halogen, Hydroxy, Alkyl, Alkoxy und -C(O)-O-Alkyl;
$R_1$ -$C_{1-3}$-Alkylene-O-$C_{1-3}$-Alkyl ist;
$R_2$ eine $C_{2-18}$-Alkylengruppe oder $C_{2-18}$-Alkenylengruppe ist, die gegebenenfalls durch ein oder mehrere nicht-peroxidische -O-Atome unterbrochen ist;
Y N oder NH ist;
Linker eine heterobifunktionelle Vernetzungsgruppe ist, die abgeleitet ist von:

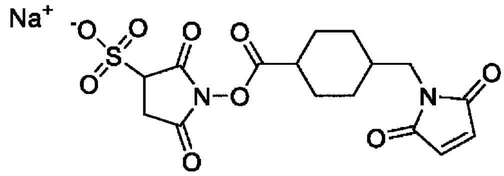

,

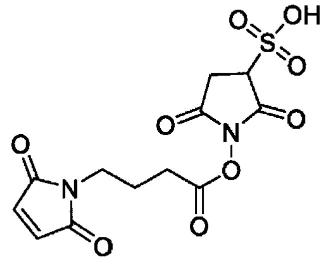

,

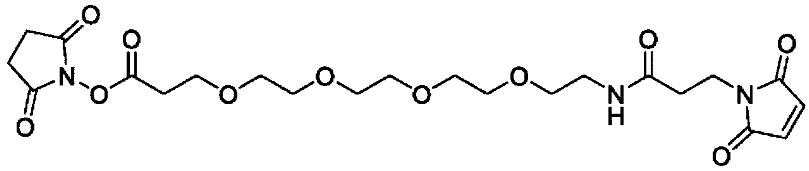

,

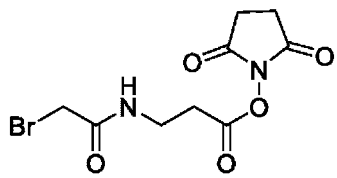

,

oder

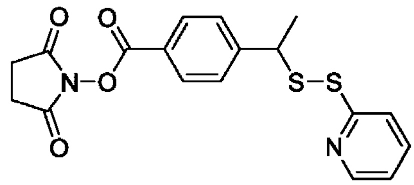

;

m = 0 oder 1; und
Z ein Antikörper ist.

**9.** Das IRM-enthaltende Konjugat nach Anspruch 8, wobei das IRM-enthaltende Konjugat die Formel (V-B) aufweist:

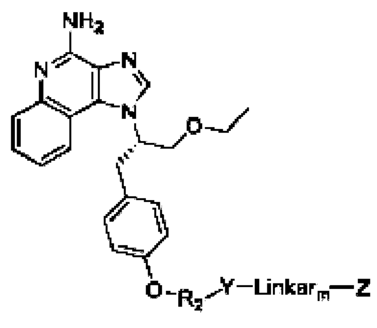

(V-B)

wobei:

Y NH ist;
m = 1.

**10.** Das IRM-enthaltende Konjugat nach Anspruch 8 oder 9, wobei $R_2$ eine $C_{2\text{-}6}$-Alkylengruppe ist.

11. Das IRM-enthaltende Konjugat nach einem der Ansprüche 8 bis 10, wobei n 0 ist.

12. Das IRM-enthaltende Konjugat nach einem der Ansprüche 8 bis 11, wobei der Linker abgeleitet ist von:

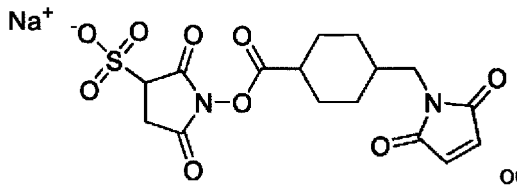

oder

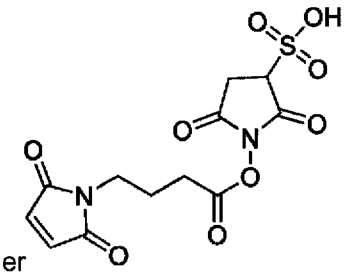

.

13. Eine pharmazeutische Zusammensetzung, umfassend die Verbindung nach einem der Ansprüche 1 bis 8 und einen pharmazeutisch verträglichen Träger.

14. Eine pharmazeutische Zusammensetzung, umfassend das IRMenthaltende Konjugat nach einem der Ansprüche 9 bis 12 und einen pharmazeutisch verträglichen Träger.

15. Die pharmazeutische Zusammensetzung nach Anspruch 13 oder 14 zur Verwendung beim Induzieren einer Cytokin-Biosynthese in einem Menschen oder Tier.

## Revendications

1. Composé de formule (I), ou sel de celui-ci :

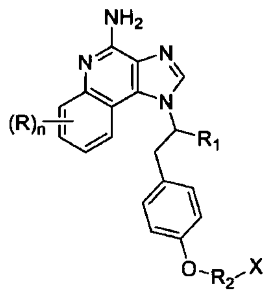

(I)

dans lequel :

n représente un nombre entier de 0 ou de 1 ;
R est choisi dans le groupe constitué d'halogène, hydroxy, alkyle, alcoxy et -C(O)-O-alkyle ;
$R_1$ représente -alkylène en $C_{1-3}$-O-alkyle en $C_{1-3}$ ;
$R_2$ représente un groupe alkylène en $C_{2-18}$ ou un groupe alcénylène en $C_{2-18}$, éventuellement interrompu par un ou plusieurs atomes -O- non peroxydiques ; et
X représente $NH_2$.

2. Composé ou sel selon la revendication 1, dans lequel R est choisi dans le groupe constitué d'halogène, hydroxy, -alkyle en $C_{1-7}$, et -alcoxy en $C_{1-7}$.

3. Composé ou sel selon la revendication 1, dans lequel n représente 0.

4. Composé ou sel selon l'une quelconque des revendications 1 à 3, dans lequel $R_2$ représente un groupe alkylène en $C_{2-18}$ éventuellement interrompu par un ou plusieurs atomes -O- non peroxydiques.

5. Composé ou sel selon l'une quelconque des revendications 1 à 4, dans lequel $R_2$ représente un groupe alkylène en

$C_{2-6}$.

**6.** Composé ou sel selon l'une quelconque des revendications 1 à 5, dans lequel le composé présente la formule (II), ou est un sel du composé de formule (II) :

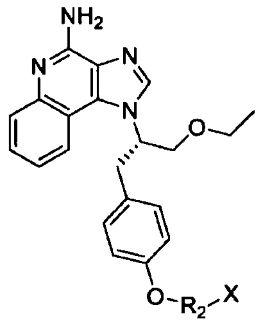

(II).

**7.** Composé ou sel selon l'une quelconque des revendications 1 à 6, représenté par la formule (IV) ou un sel de celui-ci :

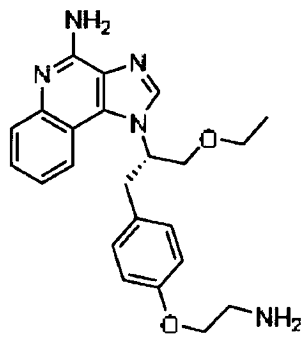

(IV).

**8.** Conjugué contenant un MRB de formule (V-A) :

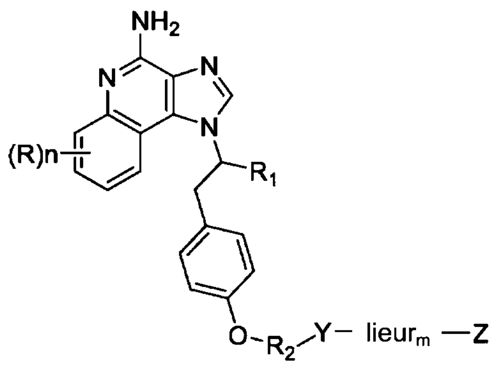

(V-A)

dans lequel :

n représente un nombre entier de 0 ou de 1 ;
R est choisi dans le groupe constitué d'halogène, hydroxy, alkyle, alcoxy et -C(O)-O-alkyle ;
$R_1$ représente -alkylène en $C_{1-3}$-O-alkyle en $C_{1-3}$ ;
$R_2$ représente un groupe alkylène en $C_{2-18}$ ou un groupe alcénylène en $C_{2-18}$, éventuellement interrompu par un ou plusieurs atomes -O- non peroxydiques ;

Y représente N ou NH ;
lieur représente un groupe de réticulation hétérobifonctionnel dérivé de :

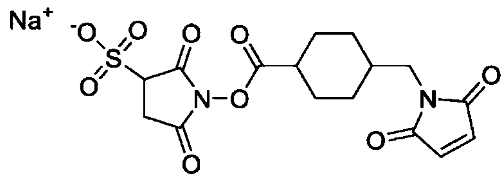

,

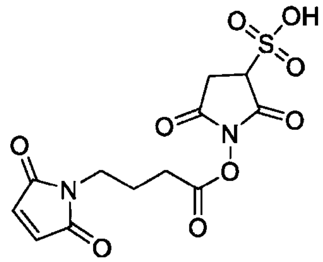

,

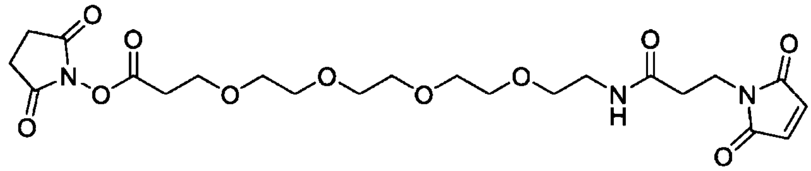

,

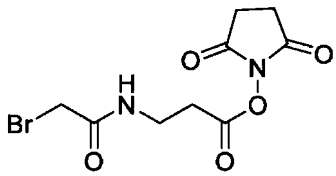

,

ou

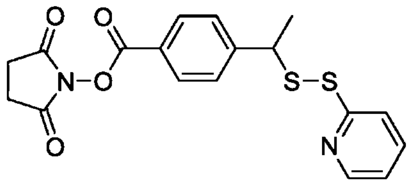

;

m = 0 ou 1 ; et
Z représente un anticorps.

**9.** Conjugué contenant un MRB selon la revendication 8, dans lequel le conjugué contenant un MRB présente la formule (V-B) :

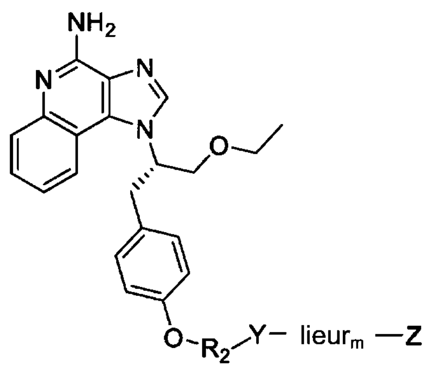

(V-B)

dans lequel :

Y représente NH ;
m = 1.

**10.** Conjugué contenant un MRB selon la revendication 8 ou 9, dans lequel $R_2$ représente un groupe alkylène en $C_{2-6}$.

**11.** Conjugué contenant un MRB selon l'une quelconque des revendications 8 à 10, dans lequel n représente 0.

**12.** Conjugué contenant un MRB selon l'une quelconque des revendications 8 à 11, le lieur étant dérivé de :

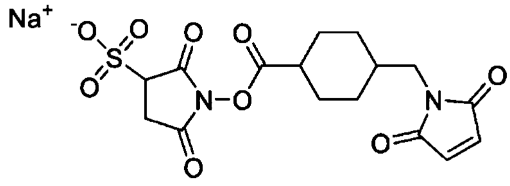

ou

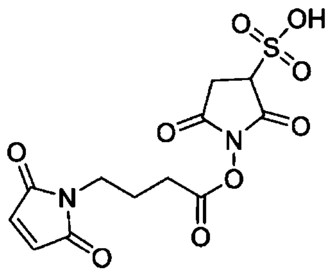

.

**13.** Composition pharmaceutique comprenant le composé selon l'une quelconque des revendications 1 à 8 et un support pharmaceutiquement acceptable.

**14.** Composition pharmaceutique comprenant le conjugué contenant un MRB selon l'une quelconque des revendications 9 à 12 et un support pharmaceutiquement acceptable.

**15.** Composition pharmaceutique selon la revendication 13 ou 14 destinée à être utilisée dans l'induction de la biosynthèse de cytokines chez un humain ou un animal.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3700674 A, Diehl
- US 5389640 A, Gerster
- US 6110929 A, Gerster
- US 7923560 B, Wightman
- WO 2005082023 A
- WO 2005110013 A
- US 2004091491 A, Kedl
- US 20060142202 A, Alkan
- WO 2019166937 A


### Non-patent literature cited in the description


- **BERGE, STEPHEN M.** Pharmaceutical Salts. *Journal of Pharmaceutical Sciences*, 1977, vol. 66, 1-19
- Remington's Pharmaceutical Sciences. Mack Publishing Co., 1990
- **LOUIS F. FIESER** ; **MARY FIESER**. Reagents for Organic Synthesis. Wiley, vol. 1-26
- **ALAN R. KATRITSKY** ; **OTTO METH-COHN** ; **CHARLES W. REES**. Comprehensive Organic Functional Group Transformations. Pergamon Press, 1995, vol. 1-6
- **BARRY M. TROST** ; **IAN FLEMING**. Comprehensive Organic Synthesis. Pergamon Press, 1991, vol. 1-8
- Beilsteins Handbuch der Organischen Chemie. Springer-Verlag
- **P.G.M. WUTS**. Greene's Protective Groups in Organic Synthesis. John Wiley & Sons, 2014, vol. 20
- Heterobifunctional Cross-Linkers. **HERMANSON, G.** Bioconjugate Techniques. Academic Press, 1996, 229
- Heterobifunctional Cross-Linkers. **HERMANSON, G.** Bioconjugate Techniques. Academic Press, 1996, 229-285
- **TOKI, B. E. et al.** *J. Org. Chem.*, 2002, vol. 67, 1866-1872
- **JEFFREY, S. C. et al.** *J. Med. Chem.*, 2005, vol. 48, 1344-1358
- **SUN, M. M. C. et al.** *Bioconjugate Chem.*, 2005, vol. 16, 1282-1290
- **TSUCHIKAMA, K.** ; **AN, Z.** *Protein Cell*, 2018, vol. 9, 33-46
- **BRAUN**. *G. Org. Synth.*, 1932, vol. 1, 431